# EUROPEAN PATENT SPECIFICATION

(11) **EP 2 416 783 B1**
(45) Date of publication and mention of the grant of the patent: **07.11.2018**
(21) Application number: 10712907.4
(22) Date of filing: 07.04.2010
(51) Int. Cl.: A61K 47/38, A61K 9/28, A61K 31/573, A61P 5/44

(54) **IMPROVED GLUCOCORTICOID THERAPY**
VERBESSERTE GLUCOKORTIKOID-THERAPIE
AMÉLIORATION DE LA THÉRAPIE PAR LES GLUCOCORTICOÏDES

(30) Priority: 07.04.2009 DK 200900470
(43) Date of publication of application: 15.02.2012
(73) Proprietor: Shire Viropharma Incorporated, Lexington, MA 02421 (US)
(72) Inventor: LENNERNÄS, Hans, S-756 52 Uppsala (SE); JOHNSSON, Jörgen, S-254 33 Helsingborg (SE); HEDNER, Thomas, S-520 10 Gällstad (SE); JOHANNSSON, Gudmundur, 302 40 Halmstad (SE); SKRTIC, Stanko, S-413 20 Göteborg (SE)
(74) Representative: Goodfellow, Hugh Robin
(86) International application number: PCT/EP2010/002178
(87) International publication number: WO 2010/115615

(56) References cited:
- WO-A-98/48782
- WO-A-03/015793
- WO-A-2005/065692
- WO-A-2005/102271
- WO-A-2008/065386
- GB-A- 1 458 676
- US-A- 6 156 743
- DEBONO MIGUEL ET AL: "Inadequacies of glucocorticoid replacement and improvements by physiological circadian therapy." EUROPEAN JOURNAL OF ENDOCRINOLOGY / EUROPEAN FEDERATION OF ENDOCRINE SOCIETIES MAY 2009, vol. 160, no. 5, May 2009 (2009-05), pages 719-729, XP002545366 ISSN: 1479-683X
- DEBONO M ET AL: "Novel strategies for hydrocortisone replacement." BEST PRACTICE & RESEARCH. CLINICAL ENDOCRINOLOGY & METABOLISM APR 2009, vol. 23, no. 2, April 2009 (2009-04), pages 221-232, XP002545367 ISSN: 1532-1908
- XU J ET AL: "Assessment of the impact of dosing time on the pharmacokinetics/ pharmacodynamics of prednisolone" AAPS JOURNAL 200806 US, vol. 10, no. 2, June 2008 (2008-06), pages 331-341, XP008111796 ISSN: 1550-7416
- BOLT H M: "Principles of circadian and alternative therapy with corticosteroids and the influence upon pituitary-adrenal system" ALLERGOLOGIE 1980 DE, vol. 3, no. 4, 1980, pages 171-176, XP008111754 ISSN: 0344-5062

## Description

### Field of the invention

The present invention relates to improved glucocorticoid therapy and to treatment or prevention of a number of disorders that are due to a diminished or disrupted endogenous glucocorticoid secretory pattern as defined in the claims. The invention is based on the findings that producing a specific serum cortisol time profile that mimics the circadian rhythm of cortisol of a healthy subject in a subject suffering from a diminished or disrupted glucocorticoid secretory pattern gives benefits with respect to reduction of side-effects.

### Introduction

Glucocorticoids (GC) are important steroids for intermediary metabolism, immune function, musculoskeletal function, connective tissue and brain function. GC deficiency occurs in adrenal insufficiency (Al) which can be primary (Addison's disease), secondary (central) due to hypopituitarism.

The outcome of GC replacement therapy has been considered satisfactory (1) until recently. Patients with hypopituitarism have double the standardised mortality rate (SMR) (2, 3) and young adults with hypopituitarism and concomitant Al have more than 7-fold the expected mortality rate (4). Moreover, patients with Addison's disease have also been shown to have more than doubled the SMR (5, 6). A possible explanation for this increased mortality rate is an inappropriate GC replacement therapy; that is, both too high maintenance doses and inadequate glucocorticoid exposure in response to stress and concurrent illnesses. Thorough re-evaluations of patients receiving GC replacement therapy have revealed that doses are too high and can be reduced in a majority of patients (7). It is likely that many patients are receiving overly high oral doses delivered with an unphysiological plasma concentration-time profile.

Pattern of delivery of conventional hydrocortisone tablets have been shown to affect well-being and also if hydrocortisone is delivered with an infusion pump in order to mimic the physiological circadian cortisol profile well being is improved (10, 11).

The estimated daily cortisol production rate in normal subjects varies in recent studies between 9 and 11 mg/m² per day (corresponding to approximately 15.5-19 mg per day in an average adult subject) (14, 15). This is much lower than the replacement dose of hydrocortisone currently being used (16, 17). Studies have therefore been performed with the objective to see the metabolic effects of reducing the daily dose of hydrocortisone but delivered in the same pattern with conventional hydrocortisone tablets. In a study performed to optimise dose of hydrocortisone 24/32 patients required a dose reduction from a mean dose of 29.5 ± 1.2 mg (SEM) to 20.8 ± 1.0 mg and 18/32 required a change in dose regimen (BID to TID) (18). After 2.3 to 6.8 months re-evaluation demonstrated an increase in serum concentration of osteocalcin, a bone formation marker (16.7 to 19.9 pg/L, p<0.01). In another carefully performed trial, effects of a dose reduction from 30 to 15 mg of daily hydrocortisone on cardiovascular function was studied (19). Before dose reduction and after 3 months of the reduced hydrocortisone dose 24-hour ambulatory blood pressure, erect and supine blood pressure, echocardiography, forearm pletysmography and cardiovascular reflexes in response to tilt, valsalva and isometric hand grip were studied. The dose reduction was well tolerated, but there were no change in body weight or any of the cardiovascular functional variables studied. Another small study reduced the dose of hydrocortisone on average by 50% in 11 patients with secondary adrenal insufficiency (20). Of some concern in this trial was a dose reduction to 10 per day of hydrocortisone in 4 out of 11 patients, which could have been too low. Also the patients included were obese with an average BMI greater than 30 kg/m². During a follow-up of 6-12 months later body weight had reduced by on average 7.1 kg and BMI had reduced from 31.5 ± 1.1 to 29.4 ±1.0 kg/m². Fasting glucose and insulin levels were unaffected by this large reduction in daily hydrocortisone replacement dose. Studies that have reduced the daily dose of hydrocortisone ranging from 30-50% without changing pattern of delivery may affect body weight and bone metabolism, but apparently does not affect blood pressure or glucose metabolism (12, 13).

### Description of the invention

There is still a need for developing glucocorticoid regimens with improved side-effect patterns. The present invention is a further development of the Applicant's proprietary technology described in WO 2005/102271, WO 2005/102271 describes pharmaceutical compositions that contain i) a part of a glucocorticoid for immediate release and ii) a part of a glucocorticoid for extended release. The composition is intended to be administered by the oral route in the morning and to mimic the circadian rhythm of cortisol in the plasma. Especially compositions are described that contain 15-50% w/w of the glucocorticoid content in the immediate release part and the remaining part in the extended release part of the composition. A clinical trial with such a composition has surprisingly revealed that a substantial reduction in side-effects is obtained if a dosage regimen is used that mimic the circadian rhythm of cortisol. Accordingly, besides a more compliant regimen further advantages are obtained cf. below. Moreover, the results strongly indicate that disturbances of the circadian rhythm (e.g. transient disturbances) of cortisol, such as e.g. stress conditions etc. may be alleviated or eliminated by re-establishing the normal circadian rhythm of the subject by administering to the subject a composition that lead to a plasma cortisol concentration-time profile that mimics that of a normal healthy subject.

The present invention provides a dosage form for use in a method of treating a subject suffering from a diminished or disrupted endogenous glucocorticoid secretory pattern according to claim 1. The present invention provides a method for reducing one or more side-effects of glucocorticoid therapy, the method comprising administering to a subject with diminished or disrupted endogenous glucocorticoid secretory pattern effective amount of hydrocortisone. More specifically, the dosing of the glucocorticoid and the delivery system employed must ensure that the circadian plasma concentration-time profile of cortisol after administration of one or more glucocorticoids mimics that of a healthy subject. As seen from the Examples herein and the definition below, the term "mimic" means that the following cortisol profile target criteria are fulfilled at least for 40% or more of the subjects tested, preferably for 50% or more of the population tested:
Criteria 1: clinically significant plasma cortisol concentration (>200 nmol/L) within 30 minutes after administration (i.e. within 6.30 am when dose administration is at 6 am)
Criteria 2: A maximal plasma concentration in the range 500-800 nmol/L within 4 hours after administration (i.e. within 10 am)
Criteria 3: Plasma concentration of 50-200 nmol/L at 12-18 hours after administration (i.e. from 6 pm to 12 pm)
Criteria 4: Plasma concentrations below 50 nmol/L at 18-24 hours after administration (i.e. from 0 am to 6 am).

In general, criteria 1 is fulfilled for 80% or more of the subjects tested, such as 90% or more, 95% or more or all of the subjects tested. Criteria 2 is normally fulfilled for 65% or more of the subjects tested, such as 75% or more, 80% or more or 85% or more of the subjects tested. Criteria 3 is generally fulfilled for 75% or more of the subjects tested, such as 85% or more, 90% or more or 95% or more of the subjects tested. Criteria 4 is more difficult to fulfill as the present study has shown that 1/6-1/7 of chronic adrenal insufficiency patients have low levels of residual cortisol production. Generally, criteria 4 is fulfilled for 40% or more of the subjects tested, such as 45% or more, 50% or more, 55% or more or 60% or more.

The present invention is based on an observed link between re-establishing the normal circadian rhythm of cortisol and reduction of the side-effects that normally are seen with glucocorticoid therapy. Accordingly, although the clinical studies reported herein are based on a specific pharmaceutical formulation for oral administration once daily, it is envisaged that any glucocorticoid formulation that makes it possible to re-establish the normal circadian rhythm of cortisol has the advantages claimed. Accordingly, the administration route may be different from the oral route, and the clock time for administration may also be different from 6 am provided that the criteria set forth above are fulfilled with respect to clock times.

Moreover, the following definitions are used herein.

### Definitions

The term *"reduction of side-effect(s)"* is intended to relate to a comparison with best standard therapy. The reduction of side effects may be observed as a reduction in the severity of a specific side-effect and/or it may be observed as reduction in the incidence and prevalence of side-effects. More specifically, side-effects relating to excess glucocorticoids include weight gain with an increase in fat mass with predominantly an abdominal distribution. Accordingly, a reduction in this specific side-effect can be expressed as less increase in weight gain or less increase in fat mass. Moreover, increased blood pressure and impaired glucose metabolism is associated with excess glucocorticoid exposure. Reduced bone mineral content and bone mineral density is also associated with excess glucocorticoids, which is seen together with reduced serum bone formation markers.

The term *"standard therapy"* as used in the present context is intended to denote a therapy involving oral administration three times daily of a glucocorticoid-containing composition, wherein the composition is a conventional tablet composition with immediate release of the glucocorticoid. Accordingly, the term "standard therapy" does not include treatment with e.g. a controlled release composition or treatment with e.g. a combination of a controlled release composition and an immediate release composition. With hydrocortisone as an example, standard therapy is suitable carried out using Cortef® (Pfizer), Hydrocorton ® (Merck), Hydrocortisone (generic e.g. MSD, Nycomed, Teva, Auden McKenzie).

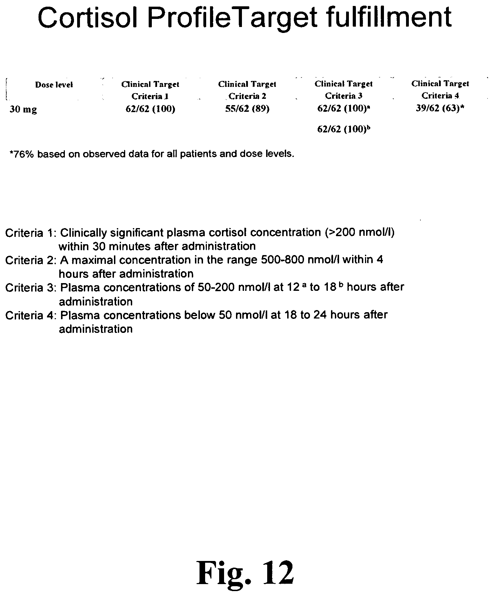

The term *"diminished or disrupted glucocorticoid secretory pattern"* is well-defined in the situation of primary, secondary and tertiary adrenal insufficiency where peak morning plasma cortisol levels are below the normal range. Patients with adrenal insufficiency also have a reduced peak plasma cortisol response to a stimulation test such as the insulin tolerance test and the short Synacten ® (synthetic adrenocorticotropic hormone) stimulation test. The glucocorticoid secretory pattern is disrupted when the peak morning plasma cortisol levels are reduced and/or the through levels around midnight are elevated leading to a less marked difference between the highest peak and the lowest through over 24 hours i.e. a flattened circadian variation as compared with what is seen in healthy subjects (see Fig. 12).

The term *"glucocorticoid"* means any steroid or steroid analog the can bind and activate the glucocorticoid receptor both through its genomic and non-genomic pathway.

The term *"circadian plasma concentration of cortisol"* means that the cortisol concentration during day and night follows that of a healthy subject measured using an immunoassay in either serum or plasma.

| Clock time | Plasma concentration of cortisol |
|---|---|
| 6 - 6.30 am | >200 nmol/L |
| 6 am - 10 am | 200 - 500 nmol/L |
| 6 pm - 12 pm | 50 - 200 nmol/L |
| 0 am - 6 am | < 50 nmol/L |

The term *"mimics"* means that the specified regimen mimics the circadian secretion pattern of cortisol and more specifically replace the daytime cortisol secretion; high morning plasma concentrations with slowly reduction in the plasma concentration throughout the day and with low nighttime plasma cortisol concentration. Accordingly, the term "mimics" has its ordinary meaning that is to resemble, simulate, approximate, follow or impersonate, but not replicate exactly or precisely. See also the discussion above.

The term *"once daily"* means that administration takes place only one time during the day. The administration may include more than one composition and more than one administration route. Preferably, the one or more oral dosage forms are administered (such as one or more single-unit tablet formulation). The daily administration time is preferably at the same time of the day (within 0.5 - 1.5 hours variation).

The term *"gain of weight"* means an increase in body weight.

The term *"increase in cardiovascular risk factors"* means an increase in or worsening of cardiovascular risk by changes in conventional risk factors such as systolic and diastolic blood pressure, lipid and carbohydrate homeostasis etc.

The term *"increase in bone degradation"* means a reduction in circulating bone formation markers and/or an increase in bone resorption marker that eventually results in loss of bone mineral content and bone mineral density leading to increased fracture risk.

The term *"metabolic risk factors"* means risks that are linked to the metabolic syndrome. The metabolic syndrome is defined by the presence of a number of clinical symptoms that include type 2 diabetes plus at least 2 of the following: Abdominal obesity, hypertriglyceridemia, low HDL cholesterol, high blood pressure or medication use, high fasting glucose or medication use for diabetes mellitus.; it is only treatable by the use of agents that treat some of these symptoms.

The term " *composite risk index"* as used herein is intended to denote a prognostic risk index for morbidity and/or mortality that is composed of several different variables ie two or more, such as those described above for cardiovascular and metabolic risk factrs but not limited to them.

The term *"statistically significant"* means that a value of p<0.05 is obtained when a statistically relevant method is used.

The term *"subject"* means a mammalian subject including dogs, cats and horses. Preferred subjects are humans.

The term *"subject with sufficient adrenal capacity"* means a subject who has not adrenal insufficiency - see above.

The term *"circadian rhythm sleep disorders"* is used accordingly to ICSD-2 classification (international classification of sleep disorders).

The term "immediate release" is generally used in accordance with the regulatory term for conventional, ordinary or plain tablets. The regulatory term for all release-controlled or release-modified tablets is "modified release". In those cases where a distinguish is made between tablets, which are conventional tablets without any release-modfying characteristics and those, which may have enhanced release characteristic, the conventional tablets are without enhanced, controlled or modified release characteristics.

The term "cortisone" includes "cortisone acetate"

As mentioned above, the present invention provides an improved glucocorticoid therapy with marked reduction in side-effects. In accordance with the clinical study reported herein, the reduction of side-effect(s) is determined by comparing 12 weeks of treatment that leads to a cortisol plasma concentration-time profile, which mimics that of a healthy subject, with 12 weeks of treatment three times daily with the same glucocorticoid in a conventional dosage form, and the total daily dose being the same in the two treatment regimens.

A conventional dosage form is typically a tablet formulation that fulfils pharmacopoeia requirements with respect to mass variation, dosage variation, disintegration, hardness etc. Thus, it is a tablet formulation that is designed to disintegrate in the stomach, to release the glucocorticoid from the disintegrated tablet thus making it readily available for absorption in the GI tract. Suitable examples include Cortef® (Pfizer), Hydrocorton ® (Merck), Hydrocortisone (generic e.g. MSD, Nycomed, Teva, Auden McKenzie).

In the examples herein are given an example, where the treatment that leads to a cortisol plasma concentration-time profile, which mimics that of a healthy subject, is a treatment once daily with an oral dosage form. Under the heading "Pharmaceutical compositions" more details are given with respect to compositions that have the desired properties with respect to mimicking the circadian rhythm of cortisol after administration.

With respect to number of subjects involved to determine a reduction of side-effects, the reduction of side-effect(s) may be determined in a test population of 12 or more subjects with diminished or disrupted endogenous glucocorticoid secretory pattern receiving said treatment that leads to a cortisol plasma concentration-time profile, which mimics that of a healthy subject, and in a comparison population of 12 or more subjects with diminished glucocorticoid secretory pattern receiving said three times daily treatment with the same glucocorticoid in a conventional dosage form, and the total daily dose being the same in the two treatment regimens.

As seen from the clinical study reported herein the benefits with respect to the following side-effects have been observed: gain of weight, increase in cardiovascular risk factors, increase in bone degradation, metabolic risk factors. The reduction of one or more side-effect(s) or a composite risk index from such factors is statistically significant. A person skilled in the art will know which statistic method to employ for a given clinical study design and how to compare the results. As appears from the examples herein, a comparison has been made with respect to test treatment and baseline and test treatment compared with conventional treatment. Baseline is at time of first randomization.

The reduction in side-effect(s) may include reduction in metabolic risk factors indicative of future risk for diabetes mellitus type 2. The metabolic risk factors may also be alterations in glucose metabolism that are known to be linked to detrimental long-term prognosis such as increased HbA1c, fasting insulin/glucose, microalbuminurea, reduced insulin sensitivity or disturbed post-prandial glucose/lipid regulation etc. The reduction in side-effect(s) may include reduction in insulin-treated diabetes mellitus (type I and / or type II) side-effects such as microvascular degeneration in eyes (macuolopathy), kidneys (microalbuminurea and consecutively progressive diabetic nephropathy), peripheral limbs (ie diabetic ulcers), heart (acute myocardial infarction, cardiomyopathies) as examples but not to be limited by. Moreover, may the improvement in metabolic risk factors in insulin-treated diabetes mellitus (type I and / or type II) consist of lower daily insulin doses, fewer insulin administrations, lower glucose fluctuations both pre and post meal and consecutively easier management of insulin-treated diabetes mellitus (type I and / or type II) and thus improved compliance, well-being. Taken all together these improvements in metabolic risk factors will also lead to reduced pharmacoeconomical cost, both direct and indirect ones.

In accordance with the results obtained in the example reported herein the metabolic risk factors may be measured as a reduction in HbA1c. Notably, the reduction may be 0.1% or more such as 0.3% or more, or 0.5% or more after 12 weeks of treatment leading to a cortisol plasma concentration, which mimics that of a healthy subject, compared with treatment three times daily with the same glucocorticoid in a conventional dosage form, and the total daily dose is the same in the two treatments.

Moreover, the reduction in side-effect(s) includes reduction in weight gain or reduction of body weight. In accordance with the results obtained in the example reported herein the weight gain after 12 weeks of treatment leading to a cortisol plasma concentration, which mimics that of a healthy subject, is reduced with at least 0.7 kg compared with treatment three times daily with the same glucocorticoid in a conventional dosage form, and the total daily dose is the same in the two treatments.

The reduction in side-effect(s) may also include reduction in cardiovascular risk factors including one or more of the systolic blood pressure and the diastolic blood pressure. In accordance with the results obtained in the example reported herein the systolic blood pressure after 12 weeks treatment is reduced with 5 mmHg or more compared with treatment three times daily with the same glucocorticoid in a conventional dosage form, and the total daily dose is the same in the two treatments. Also in accordance with the results obtained in the example reported herein the diastolic blood pressure after 12 weeks treatment is reduced with 2 mmHg or more compared with treatment three times daily with the same glucocorticoid in a conventional dosage form, and the total daily dose is the same in the two treatments.

Furthermore, the reduction in side-effect(s) may include reduction in bone degradation. In accordance with the results obtained in the example reported herein the reduction in bone degradation may be determined by measuring bone marker(s) for bone formation marker. The marker PINP may be increased with at least 5% or more, such as 7% or more, or 10% or more after 12 weeks of treatment compared with treatment three times daily with the same glucocorticoid in a conventional dosage form, and the total daily dose is the same in the two treatments.

As seen from the results of the clinical study reported herein, a marked improvement relating to reduction of side-effect(s) is seen for subjects who also suffer from diabetes mellitus such as diabetes mellitus type I or type II with or without insulin treatment. The significant large reduction in HbA1c reflects a better metabolic control in patients with diabetes mellitus. HbA1c is a strong predictor of long-term outcome in patients with diabetes mellitus.

The subject may also be at increased cardiovascular risk due to presence of cardiovascular risk factors such as hypertension, dyslipidemia, impaired glucose metabolism, renal dysfunction or cardiovascular target organ due to myocardial infarction, stroke or congestive heart failure. Especially for such subjects, a method of the present invention is advantageous due to the fact that body weight is reduced, both systolic and diastolic blood pressure is reduced and HbA1c is reduced reflecting improved glucose metabolism.

### Subjects with sufficient adrenal capacity

Benefits may also be achieved for subjects that suffer from a diminished or disrupted endogenous glucocorticoid secretory pattern. The deviation of the cortisol rhythm from the normal rhythm may be transient, i.e. it may be re-established once the disorder is alleviated. Thus, the present invention also provides a method for treating or preventing a diminished or disrupted endogenous glucocorticoid secretory pattern in a subject with sufficient adrenal capacity, the method comprising administering an effective amount of a glucocorticoid to said subject. The diminished or disrupted endogenous glucocorticoid secretory pattern is associated with disturbed circadian plasma cortisol concentration-time profile of said subject.

The disturbed circadian plasma cortisol concentration-time profile may arise from hypothyroidism, depression, sleep deprivation, insomnia, sleep disturbances, adrenal fatigue syndrome, chronic fatigue syndrome, obesity, tertiary adrenal insufficiency, circadian rhythm sleep disorders, shift-work, jet-lag, obesity, cachexia or chronic stress.

In such situations, the daily dose of said glucocorticoid (expressed as hydrocortisone equivalents) is from about 1 mg/70 kg bodyweight to about 10 mg/70 kg bodyweight, such as about 30 mg/70 kg bodyweight, such as about 5 mg/70 kg bodyweight or such as 2.5-10 mg/70 kg bodyweight. Such doses enable the circadian plasma concentration of cortisol after administration of one or more glucocorticoids to mimic that of a healthy subject.

All details and particulars mentioned under the first method aspect apply *mutatis mutandis* to this and other aspects of the present invention.

Thus, present invention relates to glucocorticoid-containing compositions for use in methods of treatment for or reduction of side-effects generally observed with glucocorticoid therapy. Moreover, the disclosure relates to use of for the manufacture of a medicament for reduction of side-effects generally observed with glucocorticoid therapy and wherein the composition is administered once daily. Consequently, the invention also enables methods for reducing side effects seen in standard glucocorticoid therapy by administration of hydrocortisone to a subject in need thereof.

### Active substance

In the present context, the term "glucocorticoid" or "glucocorticosteroid" is intended to denote a therapeutically, prophylactically and/or diagnostically active glucocorticoid or a glucocorticoid that has physiologic effect. The term is intended to include the glucocorticoid in any suitable form such as e.g. a pharmaceutically acceptable salt, complex, solvate, ester, active metabolites or prodrug thereof of in any physical form such as, e.g., in the form of crystals, amorphous or a polymorphous form or, if relevant, in any stereoisomer form including any enantiomeric or racemic form, or a combination of any of the above.

The glucocorticoid contained in a composition according to the invention is hydrocortisone. Also disclosed are compositions containing one or more glucocorticoids selected from the group consisting of hydrocortisone, cortisone, prednisolone, prednisone, methylprednisone, triamcinolone, paramethasone, betamethasone, dexamethasone, fludrocortisone, budesonide, fluticasone, cortisone acetate, and beclometasone including pharmaceutically acceptable esters, salts and complexes thereof.

As mentioned above, it is important to achieve a plasma cortisol concentration-time profile that mimics that of a healthy subject. Accordingly, the hydrocortisone is presented in a dosage form. Moreover, a part (first part) of the glucocorticoid must be released from the delivery system faster than another part (second part) in order to enable a fast appearance of the glucocorticoid in the plasma (relating to the first part) followed by a maintenance dose (extended release of the second part). The first and the second part may be presented in the same formulation or in separate formulations. In a preferred formulation, they are presented in the same formulation, notably a single-unit formulation. Moreover, if they are presented in separate formulations, the first and second formulation may be designed to be administered by the same or different administration route. These aspects are discussed further below.

It is disclosed that the one or more glucocorticoids of the first and the second part may be the same glucocorticoid or a mixture of the same glucocorticoids. Normally, this is the case as it is easy from a manufacturing point of view in those cases where both the first and the second part are parts of the same dosage form (e.g. the first and second part are contained in a tablet and the first part is provided as a coating or as a separate layer on a core containing the second part). However, in those cases where the first and second part are not part of the same dosage form (e.g. the first part is an effervescent tablet and the second part is in the form of an extended release tablet) or in those cases where an improved therapeutic result is expected when different glucocorticoids are employed, the one or more glucocorticoids of the first and the second part are different glucocorticoids or a mixture of different glucocorticoids.

As the first part of the glucocorticoid is intended for immediate release, the release and/ or absorption may take place already in the oral cavity in the case the composition is administered orally. In such cases, the glucocorticoid of choice for the first part may not be hydrocortisone (as such) or cortisone as these two active substances have a bitter taste. However, these substances may be employed provided that a sufficient taste masking is obtained. In the paragraph relating to "Pharmaceutically acceptable excipients" taste-masking is discussed in more detail. Accordingly, the one or more glucocorticoids of the first part may have an acceptable taste, may be tasteless or may be effectively taste-masked.

The glucocorticoid of the first part (as discussed above) is hydrocortisone. Further examples are synthetic glucocorticoids such as, e.g., hydrocortisone 21-succinate, prednisolone, prednisone, methylprednisone, triamcinolone, paramethasone, betamethasone, dexamethasone, fludrocortisone, budesonide, fluticasone, cortisone acetate, and beclometasone including pharmaceutically acceptable esters, salts and complexes thereof.

With respect to the second part, hydrocortisone is employed.

### Pharmaceutical compositions

The present invention provides such glucocorticoid-containing pharmaceutical compositions and kits that are designed to release a first part of the glucocorticoid relatively fast in order to enable a fast on-set of action and to release a second part of the glucocorticoid in an extended manner in order to obtain a prolonged and sustained effect of the glucocorticoid. Preferably, the compositions and kits are designed for once daily administration. The glucocorticoid in the first part is immediate released.

Accordingly, a pharmaceutical composition comprises hydrocortisone, wherein a first part of hydrocortisone is substantially immediately released and a second part of hydrocortisone is released over an extended period of time of at least about 8 hours.

Due to different potencies of the glucocorticoids, the term "hydrocortisone equivalents" has been introduced.

The term "hydrocortisone equivalents" is used herein to define the amount in mg of a specific glucocorticoid that corresponds to 1 mg of hydrocortisone for the purpose of systemic glucocorticoid therapy as generally understood by medical practitioners. The term is based on the fact that the individual glucocorticoids have different potencies and in order to achieve a desired therapeutic effect different doses of the individual glucocorticoids are required. Equivalent doses of the glucocorticoids can be calculated based on the following table.

| Glucocorticoid | Equivalent amount (mg) | Hydrocortisone equivalent (1 mg of the glucocorticoid corresponds to the listed amount in mg of hydrocortisone) |
|---|---|---|
| Cortisone acetate | 25 | 0.8 |
| Hydrocortisone | 20 | 1 |
| Prednisolone | 5 | 4 |
| Prednisone | 5 | 4 |
| Methylprednisolone | 4 | 5 |
| Triamcinolone | 4 | 5 |
| Paramethasone | 2 | 10 |
| Betamethasone | 0.75 | 26.66 |
| Dexamethasone | 0.75 | 26.66 |
| Fludrocortisone | 0.05 | 400 |

Accordingly, if the first part of the composition contains 1.5 mg betamethasone (corresponding to 40 mg hydrocortisone) and the second part of the composition contains 40 mg hydrocortisone, the total amount of hydrocortisone equivalents in the composition corresponds to 80 mg hydrocortisone. Accordingly, the first part contains 50% of the total hydrocortisone equivalents of the composition. Assuming that the total amount of the glucocorticoid in the first part is released within 1 hour in the above-mentioned dissolution test, the requirement with respect to release of the glucocorticoid from the first part within the first 45 min is that at least 25% of the total hydrocortisone equivalents are released.

The amount of the hydrocortisone of the first part, expressed as hydrocortisone equivalents, is from about 20-30% of the total hydrocortisone equivalents in the composition. This amount can be determined as the amount released 1 hour after start of testing of the composition in an in vitro dissolution test according to USP employing USP Dissolution Apparatus No. 2 (paddle), 50 rpm and simulated intestinal fluid without enzymes as dissolution medium and a temperature of 37 °C.

Normally, at least about 50% of the hydrocortisone equivalents of the first part are released within the first 45 min of the dissolution test.

A pharmaceutical composition according to the invention is suitably designed as a single composition intended for oral administration once daily. Such a composition is convenient for the patient to take and is therefore a preferred aspect. However, within the scope of the present disclosure a composition of the invention may also be a dual composition, i.e. including two different pharmaceutical forms, e.g. an extended release tablet to be ingested together with an immediate release oral pharmaceutical formulation of a glucocorticoid (or other suitable combinations). Such dual compositions are normally provided in a single package such as a kit. Accordingly, a kit may comprise
i) a first component comprising one or more glucocorticoids, the first component being designed for substantially immediately release of the one or more glucocorticoids,
ii) a second component comprising one or more glucocorticoids, the second component being designed for extended release of the one or more glucocorticoids,
wherein at least about 50% of the one or more glucocorticoids of the first component are released within the first 45 min of a dissolution test employing USP Dissolution Apparatus No. 2 (paddle), 50 rpm and simulated intestinal fluid without enzymes as dissolution medium.

In the present context the term "extended release" is intended to include all types of release which differ from the release obtained from plain tablets and that provide a release during 8 hours or more, which is a longer period of time than that obtained from plain tablets. Thus, the term includes so-called "controlled release", "modified release", "sustained release", "pulsed release", "prolonged release", "slow release", "chrono-optimized release" as well as the term "pH dependant release".

By using hydrocortisone for immediate release and hydrocortisone for extended release in specific ratios, it has been possible to mimic the circadian rhythm of cortisol after administration. Moreover, it may be envisaged that it is possible to lower the daily dosage range required to obtain a suitable therapeutic effect taking into consideration the general release profile differences in individual patients their sensitivity to the drug, and their body weights. Thus, for an average adult person, whose endogenous cortisol excretion is at a very low or zero level, the total daily dose of hydrocortisone in the range of 15-30 mg or equivalent doses of other glucocorticoids can be administered once a day in order to essentially mimic the endogenous release profile. In the present context, the term "essentially mimic" is intended to denote that the plasma profile obtained in a time period corresponding to from about 0.5-1 to about 6.5-7 hours after administration of the composition according to the invention substantially imitates or resembles the shape of the plasma profile of cortisol of a healthy subject in the morning from 6am to noon. In the case that the first and the second parts (or components in the case of a kit) of the one or more glucocorticoids are taken sequentially, the time period runs from administration of the first part.

The pharmaceutical composition or kit of the invention should provide intestinal drug absorption for about 12-18 hours after dosing.

In the following is given a detailed description of the invention relating to pharmaceutical composition. However, all details and particulars disclosed under this aspect of the invention apply *mutatis mutandis* to the other aspects of the invention. Especially, it should be noted that disclosure relating to the first and/or the second parts of a composition according to the invention also applies for a first and second component of a kit according to the disclosure.

### Pharmaceutical compositions - first and second parts

As mentioned above, a first part of the composition releases the glucocorticoid relatively quickly. For some kinds of pharmaceutical compositions it may be easy to define which part is the immediate release part (e.g. in the case of capsules containing differently colored pellets, one color for immediate release and another for extended release or in the case of a layered tablet, where the immediate release layer is on top of the extended release layer). During manufacturing of the composition it may also be relatively easy to subject the individual parts (i.e. the immediate release part and the extended release part) e.g. to in vitro dissolution test in order to evaluate the release behavior. However, with a final composition as the starting point it may in most case be difficult to define which part of the composition that is the immediate release part and which is the extended release part. Accordingly, in the present context the "immediate release part" of a composition according to the present invention is defined as the amount - expressed as hydrocortisone equivalents - released 1 hour after start of testing of the composition in an in vitro dissolution test according to USP employing USP Dissolution Apparatus No. 2 (paddle), 50 rpm or 100 rpm and simulated intestinal fluid without enzymes as dissolution medium. In contrast to known compositions without both an immediate release and extended release part i) the immediate release part contains from about 15 to about 50% of the total hydrocortisone equivalents contained in the composition, ii) at least about 50% of the hydrocortisone equivalents of the first part are released within the first 45 min of the dissolution test, and iii) the second part releases the glucocorticoid over an extended period of time of at least about 8 hours.

The *in vitro* dissolution profiles of the glucocorticoid from drug formulations according to the invention is suitably followed over time in a standardized controlled *in vitro* environment. A United States Pharmacopoeia (USP) dissolution apparatus II (paddle) coupled to automatic sampling devices and software may be used for acquiring release profiles of the drug formulations in a neutral pH environment. The dissolution profile is suitably acquired at 37 °C, 50 rpm or 100 rpm of the paddles, in a total of 300 ml or 500 ml of water. Alternatively the water can be exchanged with phosphate buffer at pH=7.0. Sampling may be performed at even time intervals such as e.g. 0, 1, 3, 5, 7, 10, 15, 20, 30, 40 50 and 60 minutes following the insertion of a pharmaceutical composition according to the invention in the dissolution medium and may be followed up to 360 min or more after insertion of a pharmaceutical composition.

However, should a comparison on a composition according to the invention be made vis-à-vis a composition not belonging to the invention the comparison regarding the dissolution test should be made using a United States Pharmacopoeia (USP) dissolution apparatus II (paddle) coupled to automatic sampling devices and software was used for acquiring release profiles of the drug formulations in a neutral pH environment. The dissolution profile is acquired at 37 °C, 50 rpm of the paddles, in a total of 300 ml of water. Sampling is performed at 0, 1, 3, 5, 7, 10 and 15 minutes following the insertion of the pharmaceutical compositions. The release profile can be followed up to 360 minutes or more in even intervals.

### Release of first part

Specific embodiments of the first part of the composition fulfil one or more of the requirements given in the following table. In general, it is preferred that the requirement stated within 30 min after start of the dissolution test is fulfilled. In preferred embodiments, at least 70% or at least 80% of the hydrocortisone equivalents contained in the first part are released within the first 30 min of the dissolution test.

| time after start of the dissolution test | % hydrocortisone equivalents released (based on the content in the first part) |
|---|---|
| within 45 min | at least about 50% such as, e.g., at least about 60%, preferably at least about 70%, at least about 80% or at least about 90% |
| preferably within 30 min | at least about 50% such as, e.g., at least about 60%, preferably at least about 70%, at least about 80% or at least about 90% |
| within 20 min | at least about 50% such as, e.g., at least about 60%, at least about 70%, at least about 80% or at least about 90% |
| within 15 min | at least about 50% |

In order to be able to obtain a relatively high plasma level of the glucocorticoids relatively fast after administration of a composition according to the invention, the amount of hydrocortisone of the immediate release part, expressed as hydrocortisone equivalents, is in a range of from about 20-30% of the total hydrocortisone equivalents in the composition.

The second part of the composition is designed to release the one or more glucocorticoids in an extended manner, i.e. the release takes place during a time period of at least about 8 hours.

In specific embodiments, the second part of one or more glucocorticoids is released over an extended period of time of at least about 10 hours. Depending on the specific formulation technique employed to prepare a composition according to the invention different release patterns can be achieved and in vivo - in vitro correlation may differ from one formulation technique to another. Accordingly, there may be situations where the *in vitro* release lasts for a much longer period of time without changing the in vivo behaviour. Accordingly, in specific embodiments the second part of one or more glucocorticoids may be released over an extended period of time of at least about 12 hours such as, e.g. at least about 15 hours or at least about 20 hours. Moreover, as long a time period as 24 hours may be of relevance in the present context.

The release mentioned above may be measured in vivo by a suitable method. Such methods are currently under development and have attracted a lot of interest. However, in general an in vitro method is preferred such as that already described herein.

### Combined release

With respect to the release of the hydrocortisone, specific embodiments of the composition fulfil one or more of the requirements given in the following table. In general, it is preferred that at least 80% or at least 90% of the hydrocortisone equivalents contained in composition are released within the first 24 hours of the dissolution test. In general the requirements described in the following table apply.

| time after start of the dissolution test | % hydrocortisone equivalents released (based on total content in the composition) |
|---|---|
| within 24 hours | at least about 80% |
| within 22 hours | at least about 80% |
| within 20 hours | at least about 80% |
| within 10 hours | at least about 50% |

However, as discussed above, there are situations where i) the release of the second part is much faster, ii) the one or more glucocorticoids is released within about 15 or 14 hours, and/or iii) the amount of the one or more glucocorticoids in the first part of the composition is relatively high. In such situations, one or more of the following requirements may apply.

| time after start of the dissolution test | % hydrocortisone equivalents released (based on total content in the composition) |
|---|---|
| within 8 hours | at least about 50% |
| within 6 hours | at least about 50% |
| within 5 hours | at least about 50% |

### Release of second part

The release of the second part of the hydrocortisone normally starts upon administration. However, there may be situations where a certain lag time is obtained, e.g. if the second part of the composition is in the form of enteric coated tablets or pellets. With respect to the release, specific embodiments fulfil one or more of the requirements are given in the following table.

| time after start of the dissolution test | % hydrocortisone equivalents released per hour (based on the content in the second part | % hydrocortisone equivalents released per hour (based on the total content of the composition) |
|---|---|---|
| from about 1 to about 8 hours | from about 3 to about 15% such as, e.g., from about 3 to about 10% | from about 1.5 to about 15% such as, e.g., from about 3 to about 15% |
| from about 1 to about 10 hours | from about 3 to about 15% such as, e.g., from about 3 to about 10% | from about 1.5 to about 15% such as, e.g., from about 3 to about 15% |
| from about 1 to about 12 hours | from about 3 to about 15% such as, e.g., from about 3 to about 10% | from about 1.5 to about 15% such as, e.g., from about 3 to about 15% |

Thus the invention relates to compositions comprising:
i) from about 3 to about 15% of the hydrocortisone equivalents contained in the second part are released per hour during a time period of from 1 to about 6 hours,
ii) from about 3 to about 10% of the hydrocortisone equivalents contained in the second part are released per hour during a time period of from about 6 to about 10 hours, and
iii) from about 3 to about 7.5% of the hydrocortisone equivalents contained in the second part are released per hour during a time period of from about 10 to about 12 hours after start of the dissolution test as defined herein.

With respect to the extended release part in principle any pharmaceutical formulation designed for extended release may be used. It is well known that the release of the active substance from some extended release formulations (e.g. matrix tablets) may be very slow especially if the release is designed as a 24-hour release. In such cases it may be necessary to estimate the total amount of hydrocortisone equivalents in the composition in order to determine the content of the second part. Accordingly, the amount of hydrocortisone equivalents of the second part of the composition may, if relevant, be determined as (Hₜₒₜₐₗ - H_{first part}), wherein Hₜₒₜₐₗ is the total amount of hydrocortisone equivalents released within 24 hours after start of the test defined in above and H_{first part} is the amount of hydrocortisone equivalents of the first part of the composition determined as defined herein.

A pharmaceutical composition according to the invention may also contain one or more bio/mucoadhesion promoting agents. Normally such bio/mucoadhesion promoting agents are present in concentration of from about 0.1 to about 25% w/w such as e.g about 1% to about 20%, such as e.g about 5% to about 15%, such as e.g. 5% to about 10% w/w. Examples of bio/mucoadhesion promoting agents include polymers including synthetic polymers, natural polymers and derivatives thereof, and mixtures thereof. The polymer may be selected from a carbomer, a polyethylene oxide, a poly co-(methylvinyl ether/maleic anhydride, and mixtures thereof; or it may be a polysaccharide. The polysaccharide may be selected from the group consisting of gelatin, sodium alginate, pectin, scleroglucan, xanthan gum; guar gum, microcrystalline cellulose, crosscaramellose, hydroxypropyl cellulose, methyl cellulose, ethyl cellulose, hydroxyethyl cellulose, hydroxypropyl cellulose, ethyl hydroxyethyl cellulose, carboxymethyl cellulose, sodium carboxymethyl cellulose, moderately cross-linked starch, and chitosan.

A pharmaceutical composition according to the invention may also contain a dissolution promoting agent.
If present, a dissolution promoting agent is present in a concentration of from about 0.05 to about 5% w/w, of the total weight of the composition such as e.g. about 0.5% to about 5%, such as e.g. about 1% to about 4%, such as e.g. 2% to about 3% w/w. The dissolution promoting agent may be selected from the group consisting of sodium lauryl sulphate, a polysorbate, a bile acid, a bile salt, a salt of cholic acid or cholanic acid, isopropyl myristate, methyl laurate, oleic acid, oleyl alcohol, glycerol monoleate, glycerol dioleate, glycerol trioleate, glycerol monostearate, glycerol monolaurate, propylene glycol monolaurate, sodium dodecyl sulfate, and a sorbitan ester.

The hydrocortisone in a composition of the invention may be present as microparticles or nanoparticles. In general, the mean particle size of such particles is 10 µm or less. Furthermore, the micro- or nanoparticles may be encapsulated such as coated with a coating comprising a lechitin or a lechitin based compound.

The composition according to the invention may also comprise a disintegrating agent. Such agents promote the dispersion of the glucucorticoids in the immediate release composition over the administration site in for example the stomach. Examples of pharmaceutically acceptable disintegrating agents are cross-linked polyvinylpyrrolidone, carboxymethyl starch, natural starch, microcrystalline cellulose, and cellulose gum. If present, it is normally used in a concentration of from about 0.5% to about %10 w/w based on the total weigh of the composition such as e.g. about 1% to about 9%, such as e.g. 2% to about 8%, such as e.g. 3% to about 7%, such as e.g. 4% to about 6%, such as about 5%.. Different pharmaceutical excipients, such as mannitol and lactose, have been found to be particularly suitable as excipients.

As mentioned above, the pharmaceutical composition according to the invention may further comprise a taste-masking agent. Examples of a taste-masking agent are e.g. menthol, peppermint, vanillin, a terpene based compound, or an artificial sweetener. In a specific embodiment, the one or more glucocorticoids are taste masked by incorporation into an inclusion complex by means of alpha-, beta-, or gamma-cyclodextrins, preferably by beta-cyclodextrins.

### Administration routes - dosages

The pharmaceutical composition of the invention may be administered by a suitable administration route, preferably the oral route. Normally the oral route is preferred due to convenience for the patient, but in the case that the first and the second part of the composition are different dosage form, the first part of the composition may suitably be designed to be administered via a mucosa in the oral cavity, the nasal cavity, the rectum, the gastrointestinal mucosa, or via pulmonary, bronchial or respiratory mucosa and epithelia.

In those cases where different dosage forms are used for the first and the second part of the composition, the final composition is normally and advantageously presented as a kit.

A preferred pharmaceutical composition is a single-unit dosage form including both the immediate release part and the extended release part. Such compositions are especially suitable for use in a long-term treatment as the composition preferably is designed to be administered once daily. However, there may be situations (e.g. due to physical activities, stress etc) where the patient may need a supplemental dose of glucocorticoid. In such situations a separate dose of the immediate release part that leads to a fast on-set can be administered to the patient.

A composition according to the present inventions aims at an administration frequency once daily. In the present context the term "once daily"/"once-a-day" is intended to mean that it is only necessary to administer the pharmaceutical composition once a day in order to obtain a suitable therapeutic and/or prophylactic response; however, any administration may comprise administration of more than one dosage unit, such as, e.g. 2-4 dosage units or different dosage units (e.g. tablets).

As mentioned above, a pharmaceutical composition of the invention is designed to be administered once daily to mimic the circadian rhythm of plasma cortisol. In order to achieve a fully physiological plasma concentration time profile of cortisol *in vivo* a significant increase from low/undetectable plasma levels of cortisol has to be achieved at approximately 4 am. This is not achievable with adequate precision with a delayed release pharmaceutical formulation administered at bedtime due to the large variation within and between individuals in gastrointestinal transit time (especially colon transit time). Hence, such a formulation with a target time for the absorption to start at 4am will result in high variability in the onset of absorption and some patients would experience high peak value in plasma earlier in the night as well as later. Therefore the present invention aims to provide the patient with a rapid absorption in order to obtain adequate and physiological plasma cortisol levels as soon as possible in the early morning. The present invention will provide a rapid absorption that will achieve clinically significant plasma concentrations of cortisol (>200 nmol/L) within 30 min. This can be achieved by an immediate release oral preparation or by parenteral transbuccal administration as demonstrated in WO 2005/102271. Moreover, a combination of an immediate release and an extended release can also be achieved by the use of a single composition such as exemplified herein. Accordingly, when the hydrocortisone is given in a composition according to the present invention, a plasma concentration-time profile is obtained that is synchronized with the biological circadian rhythms of glucocorticoid. In the present context the terms "synchronize" or "mimic" are used to denote situations where the plasma level profile of glucocorticoid after administration of a composition or kit according to the present invention has a similar shape to that of a normal healthy human subject at least for a time period corresponding to 0.5 to 6 hours after administration (i.e. if the composition or kit is administered at 6am in the morning, the plasma profile of glucocorticoid of the patient should essentially have the same shape as that of a healthy subject measured in the time period corresponding to 6.30 am to noon).

A preferred pharmaceutical composition is designed for administration once daily in the morning. Typically, the composition is administered at wake-up time, i.e. from 4am to noon, from 4am to 10am, from 4am to 9am, from 5am to 8am or from 6am to 8am, most typically at 6 to 8 o'clock in the morning. The composition is also designed to provide a 6-9 h "glucocorticoid-free" interval meaning low or undetectable plasma levels of glucocorticoid (corresponding to <50 nmol/l cortisol) late evening and night.

In general, the dosage of the hydrocortisone present in a composition according to the invention depends *inter alia* on the specific drug substance, the age and condition of the patient and of the disease to be treated.

The hydrocortisone of the first and the second part of the pharmaceutical composition should each include a hydrocortisone equivalent daily dose of 5-50 mg. For the purpose of comparison, a table is given herein describing the equivalent milligram dosage of various glucocorticoids. Normally, a pharmaceutical composition according to the present invention contains a total amount of hydrocortisone equivalents expressed as hydrocortisone in the composition from about 1 to about 80 mg. In specific embodiments, the total amount of hydrocortisone equivalents in the composition is from about 1 to about 75 mg such as, e.g., from about 1 to about 70 mg, from about 5 to about 60 mg, from about 5 to about 50 mg, from about 5 to about 40 mg or from about 10 to about 30 mg.

More specifically, normal daily dose ranges are given below

| | |
|---|---|
| Hydrocortisone | 1-30 mg |
| Cortisone | 1-20 mg |
| Betamethasone | 1-20 mg |
| Prednisolon | 1-10 mg |

| | |
|---|---|
| Dexamethasone | 0.1-2 mg |
| Fludrocortisone | 0.05-5 mg |
| Prednisone | 10-50 mg |
| Methylprednisolone | 2-20 mg |

A composition according to the invention containing a dose for once daily administration as described above is designed to provide the plasma levels described in the following table (the narrow range is the preferred range, but due to individual variations plasma level within the wider range is also satisfactory). The plasma concentrations given below are given in terms of hydrocortisone equivalents.

| Hours after administration | Plasma concentration (nmol/L) - wide | Plasma concentration (nmol/L) - narrow |
|---|---|---|
| Within the first 45 min such as, e.g. within the first 30 or 20 min | 200 or more | 200 or more |
| 2 h | 100-1000 | 400-700 |
| 6 h | 100-600 | 200-400 |
| 10 h | 50-400 | 100-300 |
| 14 h | 50-300 | 50-200 |
| 18 h | 50-100 | < 50 |

Accordingly, when the hydrocortisone is given in two different parts as single dose at the same time a plasma concentration-time profile is obtained that essentially is synchronized with the biological circadian rhythms of glucocorticoid. It is preferred that the hydrocortisone is released in manner such as to provide plasma levels as shown below:

| Hours after administration | Plasma concentration (nmol/L) |
|---|---|
| within 30 min | 200 or more |
| 2 h | 400-700 |
| 6 h | 200-400 |
| 10 h | 100-300 |
| 14 h | 50-200 |
| 18 h | < 50 |

The pharmaceutical preparations are considered as a once-daily medication to be administered at wake-up, typically at 6 o'clock to 8 o'clock in the morning. They are thus also designed to provide a 6-9 h glucocorticoid-free interval plasma levels <50 nmol/l at late evening and night, during which no extraneous glucocorticoid has to be administrated to the patient.

### Pharmaceutical dosage forms

As it appears from the above, a composition according to the invention is designed for oral administration. In the case of a kit according to the disclosure, the extended release component is suitable designed for oral administration and the immediate release part may be designed for any suitable administration route, preferably via a mucosa.
In preferred aspects, a composition according to the present invention is designed for oral administration, i.e. administration by oral intake or to the oral cavity.

Most suitably a pharmaceutical composition according to the present invention is in the form of a solid dosage form such as e.g. granules, beads, pellets and powders.

A composition according to the invention is normally presented as unit dosage forms including tablets, capsules or sachets. With respect to the immediate release part or component of a kit according to the disclosure it may be presented as a different unit dosage form including e.g. thin film for application to the oral mucosa, solutions for application via a suitable device such as, e.g., a spray to the oral or nasal mucosa, an inhaler or powder inhaler for application via pulmonary, bronchial or respiratory mucosa and epithelia, suppositories or other suitable compositions for administration to the rectal mucosa or it may be presented as immediate release tablets including chewable tablets, suckable tablets, effervescent tablets, melt tablets, lozenges, pastilles or it may be presented in a more candy-like form.

In principle any relevant formulation technique for preparing an oral controlled release composition may be applied for the extended release part of the composition. Such compositions include e.g. diffusion-controlled drug delivery systems, osmotic pressure controlled drug delivery systems, eroding drug delivery systems etc. Thus, the composition may be in the form of a single or a multiple unit dosage form intended for use as such. In the same manner, any relevant formulation technique for preparing pharmaceutical compositions may be applied when formulating the immediate release part of a composition according to the invention. A person skilled in the art of pharmaceutical formulation techniques can find guidance in the handbook Remington's Pharmaceutical Sciences and in the Example herein.

In the following is given a short review on general immediate and extended release formulation techniques with an aim of obtaining the type of dissolution profile described herein for the extended release part. In the compositions described below a person skilled in the art will know how to incorporate a part that gives rise to an immediate release of the hydrocortisone.

### Immediate release part of a composition or immediate release component of a kit according to the invention

The immediate release part comprises hydrocortisone as active substance normally together with one or more pharmaceutically acceptable excipients or carriers (herein also denoted "immediate release carrier") to provide rapid release/dissolution of the glucocorticoid *in vitro* and, after administration of the pharmaceutical composition to a patient, a rapid dissolution of the hydrocortisone at the administration site such as, e.g., in the oral cavity or in the gastrointestinal tract and a rapid absorption of the glucocorticoid *in vivo.* The one or more pharmaceutically acceptable excipients employed for the immediate release part are either inherent or they may contribute to a fast release. However, they are not intended to delay or retard the release in any manner.

The immediate release carrier comprises suitable pharmaceutical excipients and presents the hydrocortisone to the dissolution medium *in vitro* and *in vivo* in a way that provides rapid dissolution of the hydrocortisone. The immediate release part is formulated by *per se* known techniques such as for instance:
Finely divided/micronised particles of the glucocorticoid are thoroughly mixed with a water soluble pharmaceutically acceptable excipient(s) such as for instance cross-linked polyvinylpyrrolidone, carboxymethyl starch, natural starch, microcrystalline cellulose, and cellulose gum. If present, it is normally used in a concentration of from 0.5% to 10% w/w based on the total weigh of the composition, such as e.g. about 1% to about 9%, such as e.g. 2% to about 8%, such as e.g. 3% to about 7%, such as e.g. 4% to about 6%, such as about 5%. Different pharmaceutical excipients, such as mannitol and lactose, have been found to be particularly suitable as excipients. and, optionally after granulation with a suitable granulation liquid, drying and milling, optionally mixed with suitable binder(s) disintegrant(s), lubricant(s), flavouring agents, colours or other suitable agents and formed into a suitable immediate release part of the composition. The immediate release part can be formed by compression into a separate layer of a layered tablet or as the outer layer of a dry-coated tablet.

Another way to formulate the immediate release part is to first dispose a solution of the hydrocortisone onto a suitable pharmaceutical excipient(s) such as for instance lactose, mannitol or any other suitable excipient(s) and carry on as above or to first make a solid solution of the hydrocortisone in a suitable excipient such as for instance polyethylene glycol, a suitable poloxamer or any other suitable excipient and carry on as above.

The immediate release part can also be in a form of a powder mixture or a powder granulation and be mixed with an extended release part and dispensed in a capsule or a sachet. It may also be formulated into small pellets and be mixed with extended release pellets and dispensed into capsules. The mixture of the immediate release part and extended release pellets can after mixing with suitable pharmaceutical excipients to a homogeneous mixture be compressed into tablets.

The immediate release part may be formulated by coating an extended release tablet of the hydrocortisone or extended release pellets with a rapidly dissolving coating containing the hydrocortisone.

As mentioned hereinbefore, the immediate release part or component may also be a separate dosage unit such as, e.g., a mucoadhesive composition e.g. in the form of a thin film for buccal application or e.g. for application to the other oral mucosa.

It may also be in the form of a dosage form intended for administration to the nasal cavity such as, e.g., a nasal spray composition or it may be designed for rectal administration such as, e.g., a solid rectal composition as a suppository, or a semi-solid rectal composition as a rectiol or a fluid rectal composition as a rectal solution.

For administration to the pulmonary, bronchial or respiratory mucosa and epithelia the composition may be in the form of an inhaler or a powder inhaler.

### Extended release part of a composition or extended release component of a kit according to the invention

The extended release part comprises hydrocortisone as active substance in a pharmaceutically acceptable excipient or carrier (herein also denoted "extended release carrier") to provide extended release/dissolution of the glucocorticoid *in vitro* and, after administration of the pharmaceutical composition to a patient, an extended dissolution of hydrocortisone in the gastrointestinal tract and an extended absorption of the hydrocortisone *in vivo.*

The extended release carrier comprises suitable pharmaceutical excipients and presents the glucocorticoid to the dissolution medium *in vitro* and *in vivo* in a way that provides dissolution of the glucocorticoid at a suitable rate during a prolonged time period. The release kinetics may follow zero order, first order or a mixed first and zero order. Examples of different extended release technologies are e.g. single units (e.g. matrix tablets, coated matrix tablets, layered tablets, multilayer coated units etc) and multiple units (e.g. units having an extended release coating, units having an extended release matrix, units having an extended release compression coating, units having a multilayer coating etc.). In the following is given a description of general applicable extended release formulation techniques. In the compositions described below, a person skilled in the art will know how to incorporate an immediate release part that gives rise to a relatively fast release of the one or more glucocorticoids. As an example such a part may be incorporated in an outermost coating layer comprising the glucocorticoid for immediate release, it may be incorporated in a separate layer in a two- or multi-layered tablet or it may be incorporated in the form of pellets formulated without release-retarding agents. The extended release part is formulated by *per se* known techniques such as for instance:
The glucocorticoid may be embedded in a water insoluble porous matrix from which the glucocorticoid is released by diffusion through the pores. Such porous matrices can be made of insoluble plastic material, such as for instance PVC, stearic acid, paraffin or other suitable insoluble materials optionally together with suitable excipients for the formation of pores and may be present in an amount of e.g. about 0.5% to about 95%, such as e.g. about 1% to about 90%, such as about e.g. about 5% to about 85%, such as e.g. about 10% to about 80%, such as e.g. about 15% to about 75%, such as e.g. about 20% to about 70%, such as e.g. about 25% to about 65%, such as e.g. about 30% to about 60%, such as about 35% to about 55%, such as about 40% to about 50%, such as e.g. about 45% or in an amount of e.g. about 0.01% to about 10%, such as e.g. about 0.05% to about 9%, such as about e.g. about 0.1% to about 8.5%, such as e.g. about 0.15% to about 8%, such as e.g. about 0.2% to about 7.5%, such as e.g. about 0.25% to about 7%, such as e.g. about 0.3% to about 6.5%, such as e.g. about 0.35% to about 6.0%, such as about 0.4% to about 5.5%, such as about 0.45% to about 5.0%, of the total amount of the weight of the ingredients used in the process of manufacturing the composition.

Suitable porosity may be e.g. at the most about 50%, such as at the most about 45%, such as at the most about 40%, such as at the most about 35%, such as at the most about 30%, such as at the most about 25%, such as at the most about 20%, such as at the most about 15%, such as at the most about 10%, such as at the most about 5%, such as at the most about 1%.

The hydrocortisone may also be embedded in a water insoluble matrix from which the hydrocortisone is made available for dissolution by gradual erosion of the matrix. Such eroding matrices can be made of a suitable fat or of a compact of hardly soluble or insoluble pharmaceutical excipients optionally mixed with other suitable pharmaceutical excipients and may be present in an amount of e.g. about 0.5% to about 95%, such as e.g. about 1% to about 90%, such as about e.g. about 5% to about 85%, such as e.g. about 10% to about 80%, such as e.g. about 15% to about 75%, such as e.g. about 20% to about 70%, such as e.g. about 25% to about 65%, such as e.g. about 30% to about 60%, such as about 35% to about 55%, such as about 40% to about 50%, such as e.g. about 45% or in an amount of e.g. about 0.01% to about 10%, such as e.g. about 0.05% to about 9%, such as about e.g. about 0.1% to about 8.5%, such as e.g. about 0.15% to about 8%, such as e.g. about 0.2% to about 7.5%, such as e.g. about 0.25% to about 7%, such as e.g. about 0.3% to about 6.5%, such as e.g. about 0.35% to about 6.0%, such as about 0.4% to about 5.5%, such as about 0.45% to about 5.0%, of the total amount of the weight of the ingredients used in the process of manufacturing the composition.
The hydrocortisone may also be embedded in a swelling hydrophilic gel matrix from which the hydrocortisone is released by diffusion through and erosion of the matrix. Such matrices usually comprise modified cellulose material such as for instance hydroxypropyl methylcellulose in admixture with suitable pharmaceutical excipients and formulated into tablets. As examples of other suitable excipients for hydrophilic gel matrices can be mentioned by not limited to various methacrylic acid copolymers,high molecular weight polyoxyethylenes and poloxamers. and may be present in an amount of e.g. about 0.5% to about 95%, such as e.g. about 1% to about 90%, such as about e.g. about 5% to about 85%, such as e.g. about 10% to about 80%, such as e.g. about 15% to about 75%, such as e.g. about 20% to about 70%, such as e.g. about 25% to about 65%, such as e.g. about 30% to about 60%, such as about 35% to about 55%, such as about 40% to about 50%, such as e.g. about 45% or in an amount of e.g. about 0.01% to about 10%, such as e.g. about 0.05% to about 9%, such as about e.g. about 0.1% to about 8.5%, such as e.g. about 0.15% to about 8%, such as e.g. about 0.2% to about 7.5%, such as e.g. about 0.25% to about 7%, such as e.g. about 0.3% to about 6.5%, such as e.g. about 0.35% to about 6.0%, such as about 0.4% to about 5.5%, such as about 0.45% to about 5.0%, of the total amount of the weight of the ingredients used in the process of manufacturing the composition.
The hydrocortisone may also be formulated into a solid shape, such as for instance a tablet or pellet, with suitable dissolution properties and then coated with a release rate controlling membrane, such as for instance a membrane controlling the rate of diffusion of the active substance through the membrane or through pores in the membrane. Such membranes can for instance be made of ethyl cellulose or any other suitable membrane-forming excipient optionally containing a water soluble pore-forming substance such as for instance hydroxypropyl methylcellulose, sugar, sodium chloride or any other suitable water soluble substance and optionally plasticizers.

A pharmaceutical composition according to the invention may be in the form of a tablet, wherein the hydrocortisone of the first part is provided as a coating. In another specific embodiment, the hydrocortisone of first and the second part are provided as pellets, granules, beads or powders.

Accordingly, the administration means can be a formulation for oral administration of both the part for immediate release and the part for extended release. For example, the composition for oral administration can be a tablet comprising the first part (immediate release) coated outside the second part (extended release). The composition for oral administration can also be a capsule comprising the first part of the composition or components of a kit according to the disclosure.

Preferably, a composition is a single-unit dosage form for oral administration once daily. The hydrocortisone may be administered in form of an oral dosage, wherein from about 15 to about 35% w/w of the total amount of the glucocorticoids is immediate released upon administration and the remaining part of the glucocorticoids is extended released during a time period of at least about 8 hours such as at least about 12 hours. The one or more glucocorticoids is/are administered in form of a single-unit dosage form comprising a core containing a part of the glucocorticoids and the core being coated with the remaining part of the glucocorticoid. Preferably, core is of a swelling matrix type.

### Pharmaceutically acceptable excipients

In the present context the terms "pharmaceutically acceptable excipients" are intended to denote any material, which is inert in the sense that it substantially does not have any therapeutic and/or prophylactic effect *per se.* Such an excipient may be added with the purpose of making it possible to obtain a pharmaceutical, cosmetic and/or foodstuff composition, which have acceptable technical properties.

Examples of suitable excipients for use in a composition or kit according to the invention include fillers, diluents, disintegrants, binders, lubricants etc. or mixture thereof. As the individual parts of a composition or kit according to the invention are used for different purposes (e.g. immediate and extended release), the choice of excipients is normally made taken such different uses into considerations. A person skilled in the art will know which kinds of pharmaceutically acceptable excipients that are suitable choices depending on the specific dosage form in question. Other pharmaceutically acceptable excipients for suitable use are e.g. acidifying agents, alkalising agents, preservatives, antioxidants, buffering agents, chelating agents, colouring agents, complexing agents, emulsifying and/or solubilizing agents, flavours and perfumes, humectants, sweetening agents, wetting agents etc.

Examples of suitable fillers, diluents and/or binders include lactose (e.g. spray-dried lactose, α-lactose, β-lactose, Tabletose®, various grades of Pharmatose®, Microtose® or Fast-Floc®), microcrystalline cellulose (various grades of Avicel®, Elcema®, Vivacel®, Ming Tai® or Solka-Floc®), hydroxypropylcellulose, L-hydroxypropylcellulose (low substituted), hydroxypropyl methylcellulose (HPMC) (e.g. Methocel E, F and K, Metolose SH of Shin-Etsu, Ltd, such as, e.g. the 4,000 cps grades of Methocel E and Metolose 60 SH, the 4,000 cps grades of Methocel F and Metolose 65 SH, the 4,000, 15,000 and 100,000 cps grades of Methocel K; and the 4,000, 15,000, 39,000 and 100,000 grades of Metolose 90 SH), methylcellulose polymers (such as, e.g., Methocel A, Methocel A4C, Methocel A15C, Methocel A4M), hydroxyethylcellulose, sodium carboxymethylcellulose, carboxymethylene, carboxymethylhydroxyethylcellulose and other cellulose derivatives, sucrose, agarose, sorbitol, mannitol, dextrins, maltodextrins, starches or modified starches (including potato starch, maize starch and rice starch), calcium phosphate (e.g. basic calcium phosphate, calcium hydrogen phosphate, dicalcium phosphate hydrate), calcium sulfate, calcium carbonate, sodium alginate, collagen etc. Fillers, diluents or binders are e.g. calcium phosphate, dibasic dihydrate dalcium sulphate, cellulose, microcrystalline cellulose, powdered cellulose, silicified microcrystalline ethylcellulose, fumaric acid hypromellose, lactose, medium-chain triglycerides, polymethacrylates, sodium chloride, sorbitol, titanium dioxide and starch and may be present in an amount of e.g. about 0.5% to about 95%, such as e.g. about 1% to about 90%, such as about e.g. about 5% to about 85%, such as e.g. about 10% to about 80%, such as e.g. about 15% to about 75%, such as e.g. about 20% to about 70%, such as e.g. about 25% to about 65%, such as e.g. about 30% to about 60%, such as about 35% to about 55%, such as about 40% to about 50%, such as e.g. about 45% of the total amount of the weight of the ingredients used in the process of manufacturing the composition.

It is also to be understood that pharmaceutical excipients according to the invention may be present in any mix in the amounts specified herein and independently from each other in the amounts specified herein.

Specific examples of diluents are e.g. calcium carbonate, dibasic calcium phosphate, tribasic calcium phosphate, calcium sulfate, microcrystalline cellulose, powdered cellulose, dextrans, dextrin, dextrose, fructose, kaolin, lactose, mannitol, sorbitol, starch, pregelatinized starch, sucrose, sugar etc. and may be present in an amount of e.g. about 0.5% to about 95%, such as e.g. about 1% to about 90%, such as about e.g. about 5% to about 85%, such as e.g. about 10% to about 80%, such as e.g. about 15% to about 75%, such as e.g. about 20% to about 70%, such as e.g. about 25% to about 65%, such as e.g. about 30% to about 60%, such as about 35% to about 55%, such as about 40% to about 50%, such as e.g. about 45% of the total amount of the weight of the ingredients used in the process of manufacturing the composition.
Specific examples of disintegrants are e.g. alginic acid or alginates, microcrystalline cellulose, hydroxypropyl cellulose and other cellulose derivatives, croscarmellose sodium, crospovidone, polacrillin potassium, sodium starch glycolate, starch, pregelatinized starch, carboxymethyl starch (e.g. Primogel® and Explotab®) etc. and may be present in an amount of e.g. about 0.5% to about 95%, such as e.g. about 1% to about 90%, such as about e.g. about 5% to about 85%, such as e.g. about 10% to about 80%, such as e.g. about 15% to about 75%, such as e.g. about 20% to about 70%, such as e.g. about 25% to about 65%, such as e.g. about 30% to about 60%, such as about 35% to about 55%, such as about 40% to about 50%, such as e.g. about 45% or in an amount of e.g. about 0.01% to about 10%, such as e.g. about 0.05% to about 9%, such as about e.g. about 0.1% to about 8.5%, such as e.g. about 0.15% to about 8%, such as e.g. about 0.2% to about 7.5%, such as e.g. about 0.25% to about 7%, such as e.g. about 0.3% to about 6.5%, such as e.g. about 0.35% to about 6.0%, such as about 0.4% to about 5.5%, such as about 0.45% to about 5.0%, of the total amount of the weight of the ingredients used in the process of manufacturing the composition.
Specific examples of binders are e.g. acacia, alginic acid, agar, calcium carrageenan, sodium carboxymethylcellulose, microcrystalline cellulose, dextrin, ethylcellulose, gelatin, liquid glucose, guar gum, hydroxypropyl methylcellulose, methylcellulose, pectin, PEG, povidone, pregelatinized starch etc.
Glidants and lubricants may also be included in the composition. Examples include stearic acid, magnesium stearate, calcium stearate or other metallic stearate, talc, waxes and glycerides, light mineral oil, PEG, glyceryl behenate, colloidal silica, hydrogenated vegetable oils, corn starch, sodium stearyl fumarate, polyethylene glycols, alkyl sulfates, sodium benzoate, sodium acetate, talc etc. Glidants or lubricants can be present in an amount of e.g. about 0.01% to about 10%, such as e.g. about 0.05% to about 9%, such as about e.g. about 0.1% to about 8.5%, such as e.g. about 0.15% to about 8%, such as e.g. about 0.2% to about 7.5%, such as e.g. about 0.25% to about 7%, such as e.g. about 0.3% to about 6.5%, such as e.g. about 0.35% to about 6.0%, such as about 0.4% to about 5.5%, such as about 0.45% to about 5.0%, of the total amount of the weight of the ingredients used in the process of manufacturing the composition.
Other excipients which may be included in a composition or solid dosage form of the invention are e.g. flavouring agents, colouring agents, taste-masking agents, pH-adjusting agents, buffering agents, preservatives, stabilizing agents, anti-oxidants, wetting agents, humidity-adjusting agents, surface-active agents, suspending agents, absorption enhancing agents, agents for modified release, water etc. and may be present in an amount of e.g. about 0.5% to about 95%, such as e.g. about 1% to about 90%, such as about e.g. about 5% to about 85%, such as e.g. about 10% to about 80%, such as e.g. about 15% to about 75%, such as e.g. about 20% to about 70%, such as e.g. about 25% to about 65%, such as e.g. about 30% to about 60%, such as about 35% to about 55%, such as about 40% to about 50%, such as e.g. about 45% or in an amount of e.g. about 0.01% to about 10%, such as e.g. about 0.05% to about 9%, such as about e.g. about 0.1% to about 8.5%, such as e.g. about 0.15% to about 8%, such as e.g. about 0.2% to about 7.5%, such as e.g. about 0.25% to about 7%, such as e.g. about 0.3% to about 6.5%, such as e.g. about 0.35% to about 6.0%, such as about 0.4% to about 5.5%, such as about 0.45% to about 5.0%, of the total amount of the weight of the ingredients used in the process of manufacturing the composition.
The composition or kit components according to the invention may also be coated with a film coating, an enteric coating, a modified release coating, a protective coating, an anti-adhesive coating etc.

A composition according to the invention (or part thereof) may also be coated in order to obtain suitable properties e.g. with respect to extended release of the one or more glucocorticoids. The coating may also be applied as a readily soluble film containing the one or more glucocorticoids for immediate release. The coating may also be applied in order to mask any unsuitable taste of the one or more glucocorticoids. The coating may be applied on single unit dosage forms (e.g. tablets, capsules) or it may be applied on a polydepot dosage form or on its individual units.

Suitable coating materials are e.g. methylcellulose, hydroxypropylmethylcellulose, hydroxypropylcellulose, acrylic polymers, ethylcellulose, cellulose acetate phthalate, polyvinyl acetate phthalate, hydroxypropyl methylcellulose phthalate, polyvinylalcohol, sodium carboxymethylcellulose, cellulose acetate, cellulose acetate phthalate, gelatin, methacrylic acid copolymer, polyethylene glycol (Macrogol), shellac, sucrose, titanium dioxide, carnauba wax, microcrystalline wax, glyceryl monostearate, zein and may be present in an amount of e.g. about 0.5% to about 95%, such as e.g. about 1% to about 90%, such as about e.g. about 5% to about 85%, such as e.g. about 10% to about 80%, such as e.g. about 15% to about 75%, such as e.g. about 20% to about 70%, such as e.g. about 25% to about 65%, such as e.g. about 30% to about 60%, such as about 35% to about 55%, such as about 40% to about 50%, such as e.g. about 45% or in an amount of e.g. about 0.01% to about 10%, such as e.g. about 0.05% to about 9%, such as about e.g. about 0.1% to about 8.5%, such as e.g. about 0.15% to about 8%, such as e.g. about 0.2% to about 7.5%, such as e.g. about 0.25% to about 7%, such as e.g. about 0.3% to about 6.5%, such as e.g. about 0.35% to about 6.0%, such as about 0.4% to about 5.5%, such as about 0.45% to about 5.0%, of the total amount of the weight of the ingredients used in the process of manufacturing the composition or the weight of the total coating composition.

Plasticizers and other ingredients may be added in the coating material. The same or different active substance may also be added in the coating material.

The solvents used in the manufacturing process of the compositions according to present invention may be any aqueous solvent such as water optionally supplemented an alcohol such as e.g. methanol or ethanol. The solvent may also be an organic solvent such as ethanol, methanol, isopropylalcohol, dichloromethane or the likes.

Consequently, a composition according to the invention comprises (wherein the percentages are given as % of total weight of all ingredients used in the manufacture of the composition):

| Glucocorticoid | about 1.5% to about 6.5% |
|---|---|
| Polymer, Binder | about 15-25%, such as about 20% |
| Filler | about 30-40%, such as about 35% or about 37% |
| Glidant | about 0.1-0.5%, such as about 0.3% |
| Lubricant | about 0.1-0.5%, such as about 0.3% |
| Film coating system | about 1-5%, such as about 3.5% or about 4.5% |
| Solvent | about 25-40%, such as about 30% or about 35% |

It is to be understood that the solvent used in the manufacturing process of the compositions according to the invention is wholly evaporated. The compositions as mentioned above have a ratio between the first immediate release part and the second extended release part in ranges of 20-30% such as e.g. about 25% of the glucocoritcoid for the coating (first part) of the total amount of glucocorticoid and 70-80% such as e.g. about 75% of the core (second part) of the total amount of glucocorticoid. The film-former used in a coating composition may comprise polyvinyl alcohol, macrogol, talc and titanium oxide.

It is also to be clearly understood that a composition according to the invention may be composed of any combination of excipients and wherein the amounts of these excipients or may be in any interval as disclosed herein.

### Figures

When a figure shows delta changes on the Y-axis, they are absolute delta values between baseline and the stated visit (1 week, 4 weeks, 8 weeks and 12 weeks). The baseline is defined as the visit when subjects enter the randomised part of the trial i.e. after 4 weeks of run-in on trice daily (TID) conventional hydrocortisone replacement.

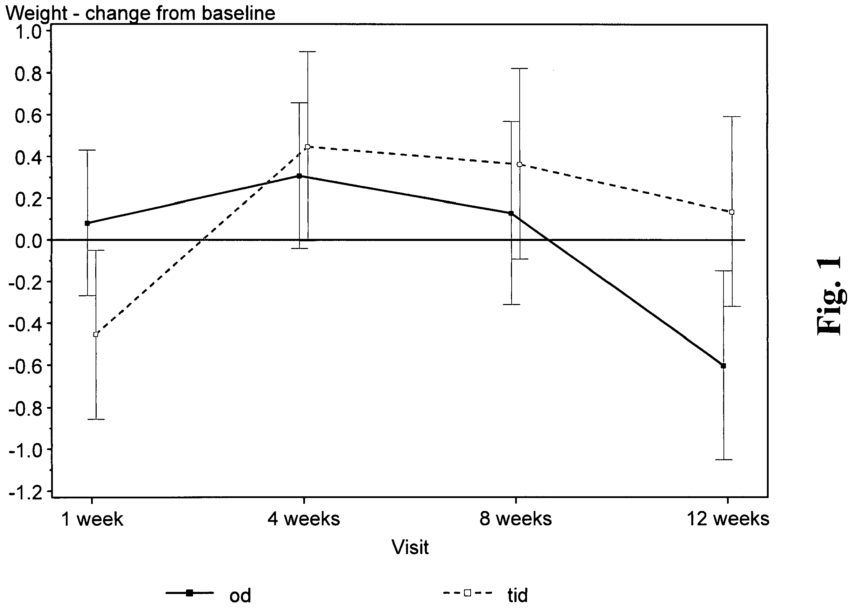

Fig. 1 shows the weight change from base line studied over the range of 1-12 weeks and the difference seen between the OD (once daily administration) and TID (three times daily administration). The figure demonstrates that the OD hydrocortisone composition confers a weight reduction of 0-6 kg after 12 weeks as compared to baseline.

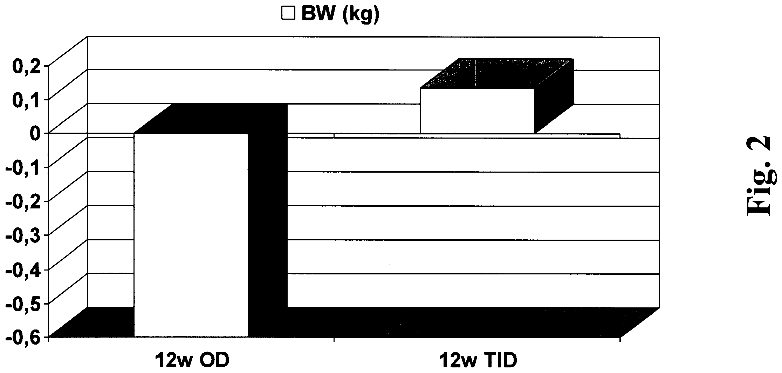

Fig. 2 shows the final difference in body weight (BW) change at 12 weeks between OD (once daily administration) and TID (three times daily administration). Note that the OD hydrocortisone composition is associated with a 0.6 kg weight reduction after 12 weeks of treatment.

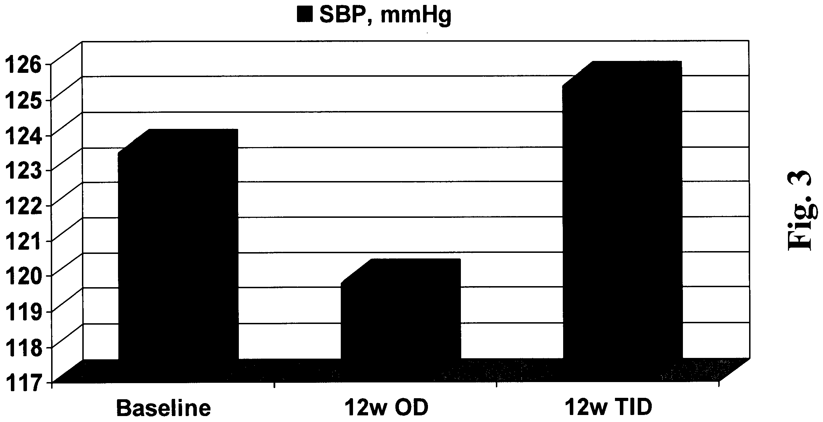

Fig. 3 shows the blood pressure in the ITT (intention to treat) study as the deviation from base line value and SBP (systolic blood pressure) between OD (once daily administration) and TID (three times daily administration). The figure demonstrates that the OD hydrocortisone composition is associated with a reduced systolic blood pressure of 5.5 mmHg as compared to the conventional TID regimen.

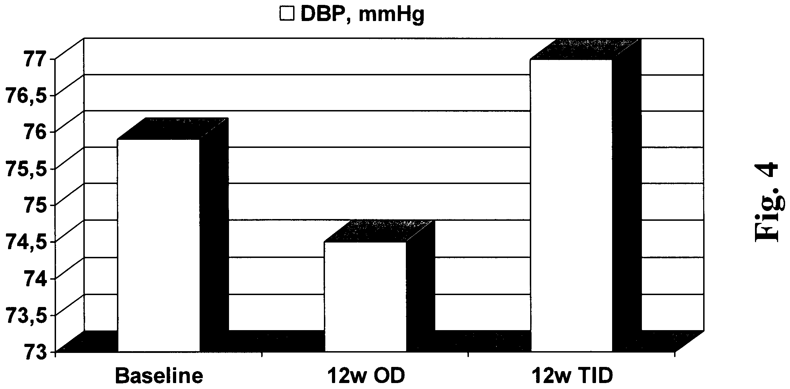

Fig. 4 shows the blood pressure in the ITT (intention to treat) study as the deviation from base line value and DBP (diastolic blood pressure) between OD (once daily administration) and TID (three times daily administration).The figure demonstrates that the OD hydrocortisone composition is associated with a reduced diastolic blood pressure of 2.3 mmHg as compared to the conventional TID regimen.

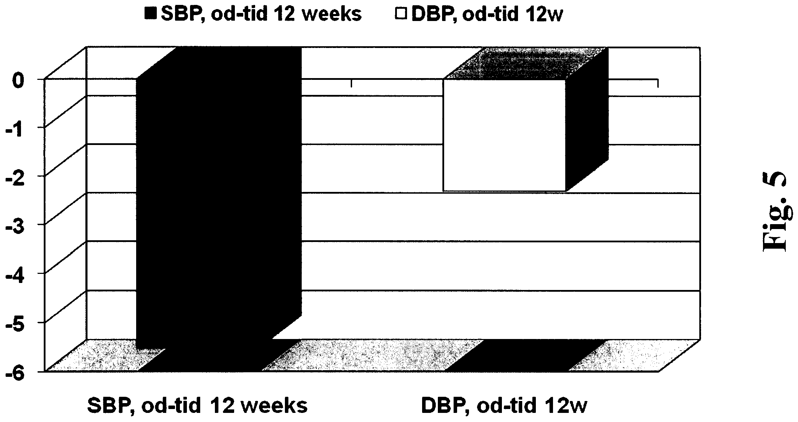

Fig. 5 shows changes in blood pressure between the OD and the TID period (Δod-Δtid). The statistics are after adjustment of the period effect.

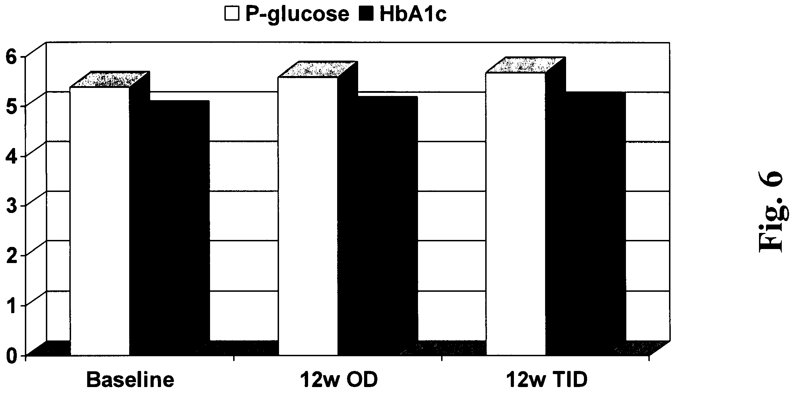

Fig. 6 shows the glucose blood concentration and the HbA1c concentration in comparison with the base line value and OD (once daily administration) and TID (three times daily administration). Note the lower HbA1c on the OD hydrocortisone composition as compared with the conventional TID regimen after 12 weeks.

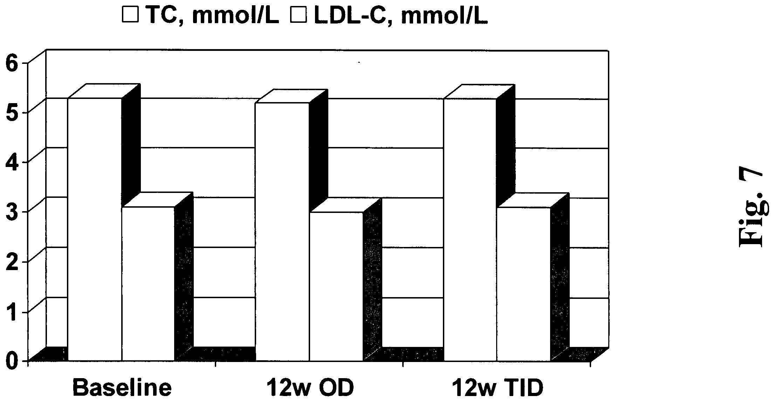

Fig. 7 show changes of TC and LDL-C concentrations after 12 weeks of treatment. Note the lower LDL-C on the OD hydrocortisone composition as compared with the conventional TID regimen after 12 weeks.

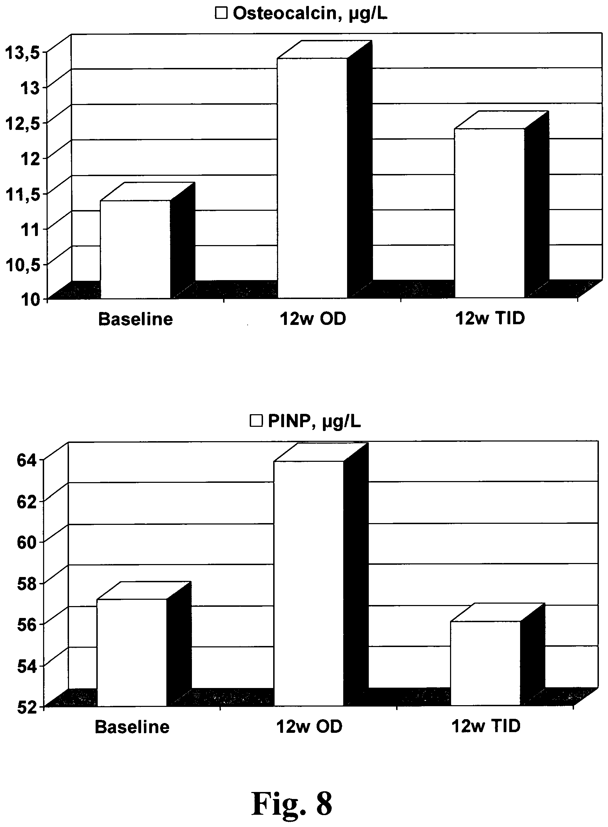

Fig. 8 shows the increase in bone resorption marker osteocalcin and bone formation marker PINP after 12 weeks in comparison with base line value and OD (once daily administration) and TID (three times daily administration). Note the higher osteocalcin and higher PINP on the OD hydrocortisone composition as compared with the conventional TID regimen after 12 weeks.

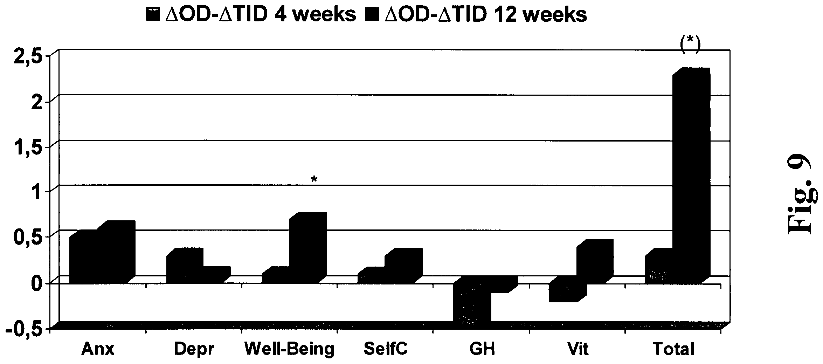

Fig. 9 shows the results from the questionnaire used to assess quality of life and the Psychological General Well-Being (PGWB) in the ITT (intention to treat) study, indicating the results from Anxiety (Anx), Depressed mood (Depr), Positive well-being (Well-Being), Self-Control (SelfC), General Health (GH), Vitality (Vit) and finally the total sum up of the assessment (Total), indicating the difference between od (once daily administration) and tid (three times daily administration). Note the improved psychological general well being (PGWB) on the OD hydrocortisone preparation as compared with the conventional TID regimen after 12 weeks.

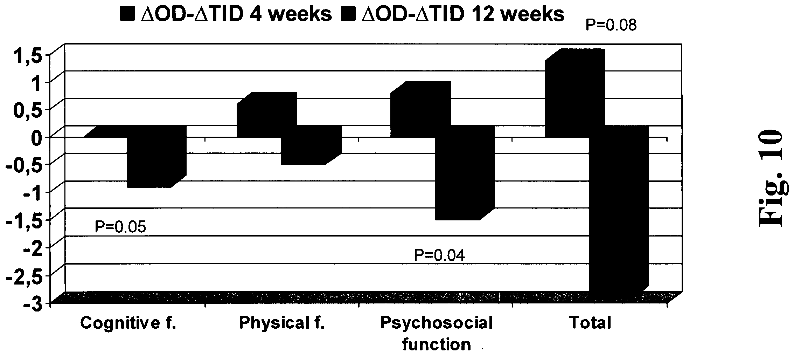

Fig. 10 shows the result of the Fatigue Impact Scale (FIS) in ITT (intention to treat). The FIS questionnaire was also used to assess fatigue and its impact on well-being as indicated in Cognitive fatigue, Physical fatigue and Psycosocial function with all factors in comparison between OD (once daily administration) and tid (three times daily administration). Note the improved (shown as lower negative scores) cognitive function, psychological function and total FIS scores on the OD hydrocortisone preparation as compared with the conventional TID regimen after 12 weeks.

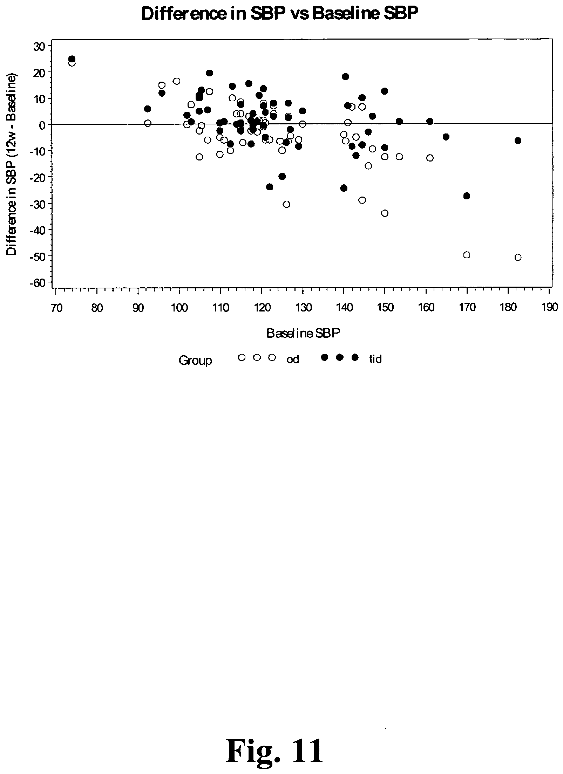

Fig. 11 shows a Bland Altman plot. Mainly patients with blood pressure in the higher range within the group of patients were the ones with the most marked reduction in blood pressure.
Fig. 12 shows the target profile criteria and fulfilment thereof for the clinical study performed and reported in the example. The results are given in the following format xx/yy (zz), wherein xx is the number of simulated patients that fulfils the criteria, yy is the total number of simulated patients, and zz is the percentage of fulfilment. The fulfilment is irrespectively of the time of the administration as all criteria are relative to the administration.

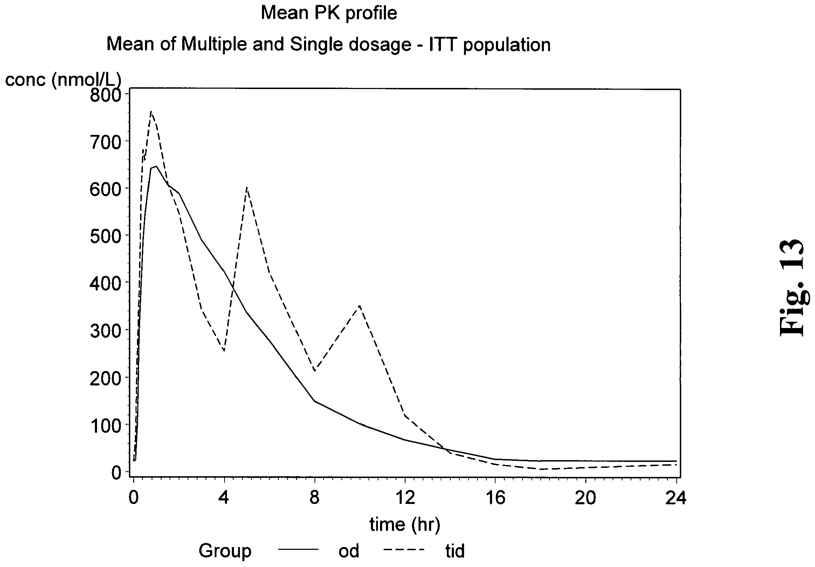

Fig. 13 shows plasma cortisol concentration-time profiles for once daily treatment and conventional treatment three times daily.

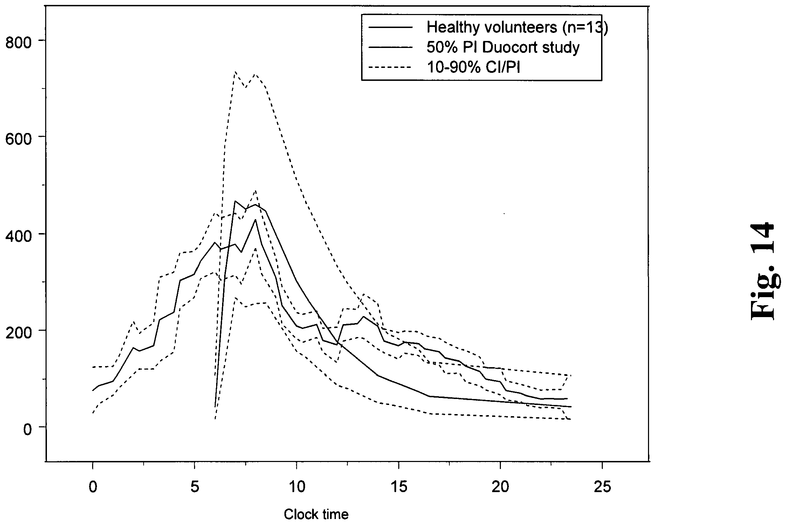

Fig. 14 shows plasma cortisol concentration-clock time profiles for healthy volunteers and for once daily treatment in accordance with the clinical study reported in the example. 50% PI is 50% prediction interval and 10-90% CI/PI is 10-90% confidence interval/prediction interval.

The invention is further illustrated in the following examples.

### Examples

### Test product

The drug product is a white, circular (diameter 8 mm), convex, modified-release tablet of two strengths, 5 mg (ER core coated with IR coating) and 20 mg (ER core coated with IR coating) hydrocortisone/tablet.
ER=extended release
IR=immediate release

The complete composition is provided in the table below.

**Composition of test product**

| **Ingredient** | **Quantity (5 mg tablet), mg/unit** | **Quantity (20 mg tablet), mg/unit** | **Standard** |
|---|---|---|---|
| Hydrocortisone | 5.0 | 20.0 | Ph.Eur. |
| Hypromellose K 100 cP (Methocel K 100) | 47.05 | 41.2 | Ph.Eur. |
| Hypromellose K 4000 cP (Methocel K4M) | 20.0 | 24.6 | Ph.Eur. |
| Cellulose, microcrystalline (Avicel PH-102) | 100.8 | 100.8 | Ph.Eur. |
| Starch, pregelatinized (Starch 1500) | 16.4 | 16.4 | Ph.Eur. |
| Silica colloidal anhydrous (Aerosil 200) | 1.0 | 1.0 | Ph.Eur. |
| Magnesium stearate | 1.0 | 1.0 | Ph.Eur. |
| Opadry II | about 13.75 | about 11.0 | Colorcon |
| Water, purified* | about 102 | about 107 | Ph.Eur. |

| | | | |
|---|---|---|---|
| ** Evaporates during the manufacturing process* | | | |

The formulations are of type swelling matrices based on hypromellose. Direct compression technique was selected.

Core tablets: Hydrocortisone, hypromellose, microcrystalline cellulose, starch pregelatinized, silica and magnesium stearate are mixed and compressed to tablets of high resistance to crushing. The tablets are circular convex tablets.

The core tablets are coated with hydrocortisone and a film former (Opadry®) to obtain hydrocortisone for immediate release. The film-former used in a coating composition may thus comprise polyvinyl alcohol, macrogol, talc and titanium oxide.
The function of each of the excipients is provided in the table, below.

**Function of excipients**

| Ingredient | Function |
|---|---|
| Hypromellose K 100 cP (Methocel K 100) | Polymer, Binder |
| Hypromellose K 4000 cP (Methocel K4M) | Polymer, Binder |
| Cellulose, microcrystalline (Avicel PH-102) | Filler |
| Starch, pregelatinized (Starch 1500) | Filler |
| Silica colloidal anhydrous (Aerosil 200) | Glidant |
| Magnesium stearate | Lubricant |
| Opadry II | Film coating system |
| Water, purified | Solvent |

### Comparison product, Hydrocortisone tablet (Hydrocortone, Merck Sharp & Dome), containing

Hydrocortisone 10 mg, (MSD).
191.1 mg Lactose Monohydrate
Magnesium stearate
Maize starch

### Clinical study

### Method

The study was conducted as an open, controlled, randomised, two-armed, two-period 12-week cross-over, multi-centre trial.

Run-in: On the first day of run-in, e.g. at inclusion, the subjects underwent a full clinical examination including medical history, physical examination, blood pressure, heart rate, ECG, routine clinical chemistry and haematology measurements. Patients on a twice a day (b.i.d.) regimen were transferred to a three times a day (t.i.d.) regimen while maintaining the same total daily hydrocortisone dose. During the 4-week run-in period, safety and tolerability of the changed regimen were assessed by telephone contact at least once and not later than 1 week prior to the next visit.

The patients were randomised to conventional t.i.d. (three times daily) or novel once daily (o.d.) therapy on the first day of study period I. Patients in study arm I then underwent standardised in-house pharmacokinetic (PK) sampling during 24 hours in order to assess single-dose PK of o.d. or t.i.d. regimen while patients in study arm II had a reduced PK sampling scheme of single dose PK on day 1-2 and returned for multiple-dose PK sampling on day 7-8.

The patients continued with the assigned treatment for 12 weeks. The patients returned every 4 weeks for study drug dispensation, adverse event (AE) assessment and collection of patient questionnaires including diurnal fatigue score. After the 12-week study period, patients were admitted for full clinical examination, laboratory sampling and other assessments including diurnal fatigue score. For patients in study arm I further reduced PK sampling took place for an initial 0-10 hours in-house and the patients returned for 24-hour samples (i.e. for multiple-dose PK) while patients in study arm II had a reduced PK sampling scheme of single dose PK on day 1-2 after cross-over and returned for multiple dose PK sampling on day 7-8. After the cross-over to the other randomised treatment (t.i.d. or novel o.d. therapy) another 24-hour in-house PK sampling (multiple dose) took place in study arm I while patients in study arm II had a reduced PK sampling scheme of single dose PK day 1-2 and the patients returned for multiple dose PK sampling on day 7-8.

The patients continued for another 12 weeks with the assigned second randomised treatment and returned every 4 weeks for study drug dispensation, AE assessment and collection of patient questionnaires including diurnal fatigue score (visual analogue scale [VAS], morning, noon, afternoon). After the 12-week study period patients were admitted for full clinical examination, laboratory sampling and other assessment including diurnal fatigue score. Patients in study arm I had another reduced PK sampling (0-10 hours in-house and a return visit for the 24-hour samples), i.e. for multiple-dose PK.

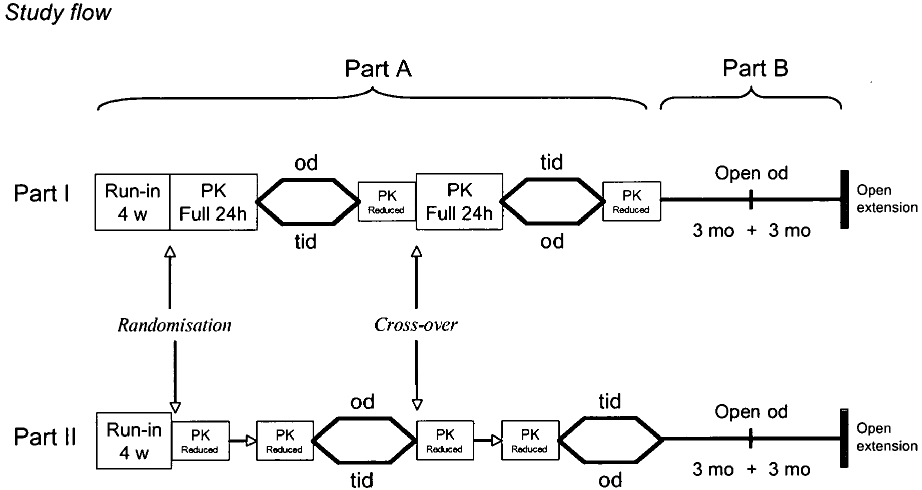

The Intention-To-Treat (ITT) population consists of all randomised patients who took at least one dose of study medication with assessments of primary efficacy variables including all PK samplings during either treatment period.

The Per-Protocol (PP) population consists of all patients in the ITT population with all protocol violators excluded.

### Demographic in the test group

The mean age of the patients was 47 years, ranging from 19 years to 71 years (ITT population). Fifty-nine per cent of the patients were males.

The mean weight was 80 kg, ranging from 54 kg to 121 kg. The mean body mass index (BMI) was 26.2 kg/m², ranging from18.6 kg/m² to 37.3 kg/m².

Mean blood pressure was 124/76 mm Hg and the mean heart rate was 66 bpm. An abnormal ECG was recorded in 7 patients (11%) at baseline.

Eleven patients (18%) were tobacco users. None of the women of fertile age had a positive pregnancy test.

At baseline, the patients' replacement doses were 20 mg (12.7%), 25 mg (9.5%), 30 mg (57.1%) or 40 mg (20.6%) per day. The daily dose at run-in was distributed as the replacement dose before study start. The replacement dose was taken either as two (55%) or three (45%) tablets per day.

Eleven (17.2%) of the patients were diagnosed with diabetes and 11 (17.5%) had a diagnosed hypertension at baseline.

### Diagnosis and main criteria for inclusion

- Males and females; age 18 years and above
- Previously diagnosed (e.g. more than 6 months ago) primary adrenal insufficiency with a stable daily glucocorticoid substitution dose for at least 3 months prior to study entry.
- An oral hydrocortisone substation therapy dose of 20, 25, 30 or 40 mg total daily dose
- Signed informed consent to participate in the study

### Duration of treatment

The duration of the study was 28 weeks in part I and 30 weeks in part II.

### Test product, dose and mode of administration

Hydrocortisone, oral tablet (modified release), 20 and 5 mg.
The hydrocortisone modified release tablet was administered orally o.d. at 8 AM in the fasting state.

During the entire trial, the run-in period and both the 12-week periods the patient's daily dose of hydrocortisone was administered as follows:

| Daily dose at run-in | Hydrocortisone modified release |
|---|---|
| 20 mg | 20+0+0 mg |
| 25 mg | (20+5)+0+0 mg |
| 30 mg | (20+5+5)+0+0 mg |
| 40 mg | (20+20)+0+0 mg |

During the run-in period, subjects received the same dose as they had at inclusion except that those on b.i.d. were switched to t.i.d.

### Reference therapy, dose and mode of administration

The reference drug (hydrocortisone, 10 mg) was administered orally t.i.d. (thrice daily; 8 AM, at 12 AM and at 4 PM). The morning dose was administered in the fasting state. During the entire trial, the run-in period, both the 12-week period the patient's daily dose of hydrocortisone was administered as follows:

| Daily dose at run-in | Reference drug t.i.d |
|---|---|
| 20 mg | 10+5+5 mg |
| 25 mg | 15+5+5 mg |
| 30 mg | 15+10+5 mg |
| 40 mg | 20+10+10 mg |

### Criteria for evaluation

### Primary Efficacy Assessment

- Difference in total S-cortisol AUC₀₋₂₄ₕ between novel combined modified release formulation and conventional thrice-daily replacement therapy for all subjects (study arm I+II)

### Secondary Efficacy Assessment

- Difference in total S-cortisol Cₛₛ, AUC_{0-∞}, C₆ₕ, C₇ₕ, T_{first}, C_{first}, Tₘₐₓ, T₂₀₀, AUC₀₋₄ₕ, AUC₄₋₁₂ₕ, AUC₆₋₁₂ₕ, AUC₁₂₋₂₄ₕ for single dose in study arm I and II and combined I+II between novel combined modified release formulation and conventional thrice-daily replacement therapy
- Difference in total S-cortisol Cₛₛ, AUC_{0-∞}, C₆ₕ, C₇ₕ, T_{first}, C_{first}, Tₘₐₓ, T₂₀₀, AUC₀₋₄ₕ, AUC₄₋₁₂ₕ, AUC₆₋₁₂ₕ, AUC₁₂₋₂₄ₕ for multiple dose in study arm I and II and combined I+II between novel combined modified release formulation and conventional thrice-daily replacement therapy
- Difference in total S-cortisol Cₛₛ, AUC_{0-∞}, T_{first}, C_{first}, Tₘₐₓ, T₂₀₀, AUC₀₋₄ₕ, AUC₄₋₁₂ₕ, AUC₆₋₁₂ₕ, AUC₁₂₋₂₄ₕ for intercurrent illness dosage regimens in study arm I and II and combined I+II between novel combined modified release formulation and conventional thrice-daily replacement therapy
- Difference in tolerability and safety between novel combined modified release formulation and conventional t.i.d. replacement therapy
- Difference in tolerability and safety between an intercurrent illness dosage regimen of the novel combined modified release formulation in comparison to the conventional t.i.d. replacement therapy.
- Difference in well-being as by SF36, Fatigue Impact Scale, Psychological General Well Being (PGWB) between novel combined modified release formulation and the conventional t.i.d. replacement therapy
- Difference in diurnal fatigue score (VAS scale) between novel combined modified release formulation and conventional t.i.d. replacement therapy
- Difference in patient preference and compliance between novel combined modified release formulation and conventional t.i.d. replacement therapy
- Difference in patient preference between an intercurrent illness dosage regimen of the novel combined modified release formulation in comparison to the conventional t.i.d. replacement therapy.
- Difference in 24-hour urinary free cortisol excretion between the novel combined modified release formulation in comparison to the conventional t.i.d. replacement therapy.

### Pharmakokinetics

In this study, the plasma plasma exposure of cortisol over time was different between o.d. treatment with a novel modified release dosage form and t.i.d. treatment using a conventional tablet. Especially the early exposure was more rapid and sustained during the first four hours after o.d. dosing than after t.i.d. dosing. This was demonstrated by similar values of Cfirst and T200 and a higher plasma exposure during the first hours (AUC0-4h). The controlled release part of the dosage form provides a sustained plasma concentration profile without any deep through values during the day. In the late afternoon, evening and night, the plasma plasma exposure of cortisol was significantly higher for the t.i.d. than for the OD treatment. Therefore in patients with primary adrenal insufficiency (Al) Compared to the conventional t.i.d. treatment compared with, the o.d. treatment resulted in a plasma plasma profile of cortisol in these Addison's Disease (AD) patients that significantly more resembled the circadian diurnal profile of cortisol in healthy individuals. Already after three months treatment, this change to a more physiologically relevant cortisol profile using the same oral dose of hydrocortisone affected variables for effect, safety and quality of life in a positive way

### Tolerability, preference, compliance and safety

Although a majority of the patients (53.4%) assessed the treatments as equally well tolerated, the patient preference questionnaire at 12 weeks showed that the benefit of o.d. treatment was assessed as large, or very large, by 85% of the patients. Over the study period, the mean ordinary cortisol dose administered was similar during o.d. (2572 mg) and t.i.d. treatment (2552 mg). The frequency and severity of adverse event was smiling among the two treatment periods demonstrating that the new o.d. treatment was equally as safe as hydrocortisone administered t.i.d.

### Study-specific Safety Parameters

In addition to collection of AEs as described above, the following parameters were collected to compare safety and tolerability of the novel combined modified release formulation in comparison to conventional therapy:
- Bone formation (osteocalcin) and bone resorption markers (PIPC)
- Glucose homeostasis (fasting plasma glucose, fasting serum insulin, HbA1c, calculated insulin resistance)
- Blood pressure (office)
- Serum electrolytes (S-Na, S-K, S-Ca)
- Serum lipid (S-TG, S-cholesterol, S-HDL, S-LDL)
- Weight

### Reduction in body weight

There was during o.d. treatment a decrease in mean body weight and BMI. This trend was not expected to be seen as early as within 3 months. The weight reduction was not a global effect, but mainly seen in those with the higher baseline weight. This also supports the above reasoning that this finding is due to a more favourable metabolic effect of the new regime rather than an effect of glucocorticoid insufficiency. Moreover, previous studies of dose reduction of glucocorticoid replacement have not been able to see weight loss. Therefore the diurnal profile produced with the o.d. treatment may be responsible for the significant weight loss seen in this study.

| | | **od** | | **tid** | | |
|---|---|---|---|---|---|---|
| | | **(n=64)** | | **(n=62)** | | |
| **Visit** | **Variable** | **Mean (SD) Median (Min; Max) n=** | **p-value within group** | **Mean (SD) Median (Min; Max) n=** | **p-value within group** | **p-value tid-od** |
| **1 week** | | n=46 | | n=46 | | |
| | **Weight - change from baseline (kg)** | 0.080 (1.209) -0.200 (-1.700; 4.100) n=46 | 0.9291 | -0.454 (1.394) -0.350 (-4.000; 2.300) n=46 | 0.0563 | 0.0534 |
| | **BMI** - **change from baseline (kg/m²)** | 0.027 (0.394) -0.058 (-0.610; 1.265) n=46 | 0.9622 | -0.151 (0.466) -0.114 (-1.487; 0.787) n=46 | 0.0556 | 0.0468 |
| | | | | | | |
| **4 weeks** | | n=63 | | n=62 | | |
| | **Weight - change from baseline (kg)** | 0.306 (1.414) 0.300 (-2.600; 3.300) n=63 | 0.1340 | 0.447 (1.789) 0.400 (-3.700; 3.800) n=60 | 0.0493 | 0.6098 |
| | **BMI - change from baseline (kg/m²)** | 0.102 (0.460) 0.108 (-0.840; 1.019) n=63 | 0.1029 | 0.152 (0.591) 0.121 (-1.338; 1.363) n=60 | 0.0451 | 0.5480 |
| | | | | | | |
| **8 weeks** | | n=63 | | n=63 | | |
| | **Weight - change from baseline (kg)** | 0.127 (1.764) 0.300 (-4.100; 4.000) n=62 | 0.4889 | 0.363 (1.838) 0.650 (-3.900; 3.800) n=62 | 0.0704 | 0.1293 |
| | **BMI - change from baseline (kg/m²)** | 0.045 (0.578) 0.103 (-1.324; 1.208) n=62 | 0.4959 | 0.123 (0.615) 0.209 (-1.379; 1.241) n=62 | 0.0623 | 0.1181 |
| | | | | | | |
| **12 weeks** | | n=62 | | n=60 | | |
| | **Weight** - **change from baseline (kg)** | -0.602 (1.815) -0.550 (-4.300; 3.300) n=62 | 0.0191 | 0.135 (1.802) 0.300 (-4.100; 3.600) n=60 | 0.4504 | 0.0025 |
| | **BMI** - **change from baseline (kg/m²)** | -0.192 (0.589) -0.183 (-1.524; 1.019) n=62 | 0.0212 | 0.056 (0.603) 0.091 (-1.488; 1.355) n=60 | 0.3383 | 0.0017 |
| For continuous variables Mean (SD) / Median (Min; Max) / n= is presented. - | | | | | | |
| For comparison over time within groups Wilcoxon Signed Rank test was used. | | | | | | |
| For comparison between groups Wilcoxon Signed Rank test was used for the difference. | | | | | | |

### Blood Pressure

The mean systolic blood pressure decreased from 123.5 mm Hg at baseline to 119.8 mm Hg at 12 weeks during o.d. treatment while it increased to 125.4 mm Hg at 12 weeks during t.i.d. treatment. The difference in change between the treatments over 12 weeks (-5.5 mm Hg) was statistically significant (p=0.0001). Similarly, the mean diastolic pressure decreased from 75.9 mm Hg at baseline to 74.5 mm Hg at 12 weeks during o.d. treatment while it increased to 77.0 mm Hg at 12 weeks during t.i.d. treatment. The difference between the treatments in change over 12 weeks (-2.3 mm Hg) was statistically significant (p=0.0343). To the contrary, the mean heart rate increased from 65.2 bpm at baseline to 67.4 bpm at 12 weeks during o.d. treatment while it decreased to 64.5 mm Hg at 12 weeks during t.i.d. treatment. The increase in heart rate during OD treatment was probably caused by the sympaticus effects of cortisone. The reduction in blood pressure is unsuspected as previously well performed trials only reducing the dose of hydrocortisone from 30 to 15 mg per day have not been able to demonstrate any effect on blood pressure (19). The only plausible explanation for these hemodynamic changes during o.d. is the change in the plasma cortisol profile. These large changes in both systolic- and diastolic blood pressure may have important impact on the long-term outcome in patients with adrenal insufficiency as current data show that this patient population has reduced life expectancy mainly due to premature cardiovascular deaths (3, 5). Moreover, the magnitude of these changes are clinically significant as they equal addition of one anti-hypertensive agent.

The improvement in blood pressure is important in this patient population since current replacement therapy with glucocorticosteroids is associated with cardiovascular diseases. The improvement in blood pressure was even more pronounced also seen in the subgroup of 13 patients with diabetes DM (difference in change in systolic blood pressure of -3.9 mm Hg and in diastolic pressure of -3.9 mm Hg, both in favour of o.d. treatment). Patient with both Addison's disease and DM have higher mortality rate that patients with Addison's disease alone (5)and hypertension in patients with DM is one of the most important factor leading to macro- and microvascular diabetes complications. An important finding is that orthostatic blood pressure or symptoms related to low blood pressure was not reported and the fact that mainly patients with blood pressure in the higher range within the group of patients were the ones with reductions (see Bland Altman plot in Figure 11). These supportive data exclude the possibility that the reduction in blood pressure was a symptom of glucocorticoid insufficiency.

| | | | **od** | | **tid** | | **od-tid** | |
|---|---|---|---|---|---|---|---|---|
| **Wee k** | **Variable** | **Baseline** | **Value** | **Change to visit** | **Value** | **Change to visit** | **Difference** | **p-value** |
| **1** | **SBP (mm Hg)** | 123.5 (19.5) 120.0 (74.0; 182.5) n=64 | 120.5 (15.3) 118.0 (86.5; 155.5) n=46 | -2.8(10.8) -1.8 (-29.0; 17.0) n=46 | 120.1 (15.9) 116.5 (88.0; 160.0) n=46 | -3.2 (11.3) -3.0 (-42.5; 17.0) n=46 | 0.3(10.5) 1.0 (-23.5; 26.0) n=46 | 0.7855 |

| | | | **od** | | **tid** | | **od-tid** | |
|---|---|---|---|---|---|---|---|---|
| **Wee k** | **Variable** | **Baseline** | **Value** | **Change to visit** | **Value** | **Change to visit** | **Difference** | **p- value** |
| | **DBP** (mm Hg) | 75.9 (11.4) 75.5 (47.5; 105.0) n=64 | 74.5 (9.7) 74.0 (48.5; 105.0) n=46 | -1.8 (7.2) -1.0 (-18.0; 22.0) n=46 | 74.5 (9.3) 74.8 (55.0; 96.5) n=46 | -1.8 (8.3) -2.5 (-22.0; 13.0) n=46 | 0.0 (7.8) 1.0 (-14.0; 22.0) n=46 | 0.8027 |
| | **Heart rate (bpm)** | 65.2 (10.5) 64.3 (47.5; 97.0) n=64 | 66.4 (11.0) 66.0 (41.5; 87.0) n=46 | 0.6 (7.4) 0.3 (-24.0; 15.0) n=46 | 65.3 (9.7) 63.0 (46.5; 94.5) n=46 | -0.6 (6.3) -0.3 (-22.0; 11.0) n=46 | 1.1 (7.0) 0.0 (-13.0; 18.0) n=46 | 0.4132 |
| **4** | **SBP (mm Hg)** | 123.5 (19.5) 120.0 (74.0; 182.5) n=64 | 122.9 (13.3) 120.0 (101.0; 156.5) n=64 | -0.6 (14.2) 0.5 (-45.5; 38.0) n=64 | 123.5 (15.3) 122.3 (97.5; 162.5) n=62 | -0.4 (13.6) 1.8 (-53.5; 31.0) n=62 | -0.9 (10.7) -0.5 (-25.0; 28.5) n=62 | 0.5301 |
| | **DBP (mm Hg)** | 75.9 (11.4) 75.5 (47.5; 105.0) n=64 | 73.2 (9.1) 72.8 (55.5; 99.0) n=64 | -2.6 (8.5) -2.0 (-21.5; 18.5) n=64 | 73.8 (11.6) 71.5 (49.0; 110.0) n=62 | -2.1 (9.5) -1.5 (-27.5; 19.0) n=62 | -0.6 (7.6) -0.5 (-19.0; 16.5) n=62 | 0.6190 |
| | **Heart rate (bpm)** | 65.2 (10.5) 64.3 (47.5; 97.0) n=64 | 72.1 (11.4) 72.0 (48.5; 96.0) n=64 | 6.9(8.4) 7.5 (-15.0; 21.5) n=64 | 68.5(10.5) 66.8 (40.5; 94.0) n=62 | 3.6 (8.1) 4.3 (-16.5; 20.0) n=62 | 3.3(7.6) 2.3 (-10.0; 24.0) n=62 | 0.0043 |
| **8** | **SBP (mm Hg)** | 123.5 (19.5) 120.0 (74.0; 182.5) n=64 | 121.2 (16.1) 120.0 (87.5; 164.5) n=64 | -2.3 (13.1) 0.5 (-56.0; 23.5) n=64 | 124.4 (16.0) 123.0 (90.0; 167.0) n=64 | 0.9 (11.2) 0.5 (-26.0; 26.0) n=64 | -3.2 (10.9) -1.5 (-40.5; 22.5) n=64 | 0.0549 |
| | **DBP (mm Hg)** | 75.9(11.4) 75.5 (47.5; 105.0) n=64 | 71.8(11.2) 71.5 (44.5; 102.0) n=64 | -4.0 (8.6) -4.0 (-24.5; 17.5) n=64 | 73.6(10.4) 74.3 (53.0; 98.0) n=64 | -2.2 (8.7) -2.0 (-26.5; 23.5) n=64 | -1.8 (8.2) -1.3 (-27.5; 13.5) n=64 | 0.2374 |
| | **Heart rate (bpm)** | 65.2 (10.5) 64.3 (47.5; 97.0) n=64 | 70.1 (10.7) 70.5 (48.5; 92.0) n=62 | 4.8 (7.7) 4.3 (-18.0; 19.5) n=62 | 70.4 (11.2) 70.5 (46.5; 105.0) n=64 | 5.2 (9.5) 4.3 (-19.0; 30.5) n=64 | -0.3 (8.2) 1.0 (-31.0; 20.0) n=62 | 0.8316 |
| **12** | **SBP (mm Hg)** | 123.5 (19.5) 120.0 (74.0; 182.5) n=64 | 119.8 (14.5) 119.0 (92.5; 160.0) n=63 | -3.8 (13.1) -2.5 (-51.0; 23.5) n=63 | 125.4 (17.7) 120.5 (98.0; 176.0) n=61 | 1.4(10.3) 1.0 (-27.5; 25.0) n=61 | -5.5(11.3) -4.0 (-44.5; 20.5) n=60 | 0.0001 |

| | | | **od** | | **tid** | | **od-tid** | |
|---|---|---|---|---|---|---|---|---|
| **Wee k** | **Variable** | **Baseline** | **Value** | **Change to visit** | **Value** | **Change to visit** | **Difference** | **p- value** |
| | **DBP (mm Hg)** | 75.9 (11.4) 75.5 (47.5; 105.0) n=64 | 74.5 (9.8) 73.0 (56.0; 111.0) n=63 | -1.4 (8.2) 0.0 (-24.0; 22.5) n=63 | 77.0 (9.5) 78.5 (54.0; 95.0) n=61 | 0.8 (7.4) 0.0 (-16.0; 18.0) n=61 | -2.3 (8.0) -1.0 (-22.0; 16.0) n=60 | 0.0343 |
| | **Heart rate (bpm)** | 65.2 (10.5) 64.3 (47.5; 97.0) n=64 | 67.4 (11.0) 67.0 (50.0; 102.0) n=63 | 2.0 (7.2) 2.5 (-21.0; 19.0) n=63 | 64.5 (10.4) 63.5 (41.0; 94.0) n=61 | -0.1 (7.3) -0.5 (-20.5; 20.0) n=61 | 2.2 (6.3) 2.8 (-19.0; 20.0) n=60 | 0.0026 |

### Glucose and HbA1c

There was a statistically significant difference between o.d. and t.i.d. treatment for the differences in change in HbA1c indicating a lower mean level of glucose over time on o.d. treatment. Only minor differences between the treatment groups were observed for other parameters on glucose metabolism (fasting plasma glucose, insulin and HOMA index). A significant proportion of patients in the study also had DM type 1 e.g. 11 and two patients had DM type 2. A post-hoc analysis of patients with diabetes DM was therefore performed showing that both fasting plasma glucose and HbA1c values levels were statistically significantly lower at 12 weeks on o.d. treatment compared to t.i.d. treatment. This was not expected to be seen as early as within 3 months and can be considered a major finding in this population of patients who have multiple daily insulin injections together with their Addison's disease. Moreover, is the magnitude in HbA1c reduction clinically significant in terms of reducing future cardiovascular morbidity and mortality. The effect in the diabetics was even larger and here the clinical benefits are shown immediately i.e. already during the study period the patients reported better management of their diabetes, lower insulin doses, fewer side-effects etc.

| | | **od** | | **tid** | | **od-tid** | |
|---|---|---|---|---|---|---|---|
| | **Baseline** | **12 weeks** | **Change to 12 weeks** | **12 weeks** | **Change to 12 weeks** | **Difference** | **P- value** |
| **GLUKOS (mmol/L)** | 5.4 (2.4) 4.7 (3.4; 14.6) n=47 | 5.6 (2.6) 4.8 (3.6; 14.6) n=61 | 0.0 (1.5) 0.1 (-6.3; 3.6) n=46 | 5.7 (2.8) 4.7 (3.8; 17.7) n=59 | -0.0 (1.1) 0.0 (-5:9; 1.6) n=44 | 0.1 (1.2) 0.1 (-7.1; 3.9) n=57 | 0.3729 |
| **HbA1c (%)** | 4.8 (1.0) 4.6 (3.8; 8.3) n=44 | 4.9 (0.9) 4.6 (3.8; 7.8) n=60 | 0.0 (0.3) 0.1 (-0.8; 1.3) n=41 | 5.0(1.1) 4.6 (4.0; 9.0) n=58 | 0.1 (0.2) 0.1 (-0.4; 0.7) n=39 | -0.1 (0.4) -0.1 (-1.9; 1.0) n=56 | 0.0008 |

Table below is on the diabetic subpopulation (11 type 1 and 2 type 2).

| | | **od** | **tid** | |
|---|---|---|---|---|
| | | **(n=64)** | **(n=62)** | |
| **Visit** | **Variable** | **Mean (SD)** | **Mean (SO)** | |
| | | **Median (Min; Max)** | **Median (Min; Max)** | **p-value** |
| | | **n=** | **n=** | **tid-od** |
| **12 weeks** | | n=13 | n=12 | |
| | **Glucose (mmol/L)** | 9.37 (4.22) | 9.66 (4.31) | 0.5332 |
| | | 9.05 (3.90; 14.60) | 9.70 (4.40; 17.70) | |
| | | n=12 | n=12 | |
| | **HbA1c (%)** | 6.11 (1.21) | 6.53 (1.58) | 0.0020 |
| | | 6.30 (4.00; 7.80) | 6.35 (4.00; 9.00) | |
| | | n=13 | n=12 | |
| For continuous variables Mean (SD) / Median (Min; Max) / n= is presented. | | | | |
| For comparison over time within groups Wilcoxon Signed Rank test was used. | | | | |
| For comparison between groups Wilcoxon Signed Rank test was used for the difference. | | | | |

### Lipids

Mean S-cholesterol and S-LDL values remained at approximately the same level as at baseline during both o.d. and t.i.d. treatment. Mean S-HDL was 1.4 mmol/l both at baseline and at 12 weeks during o.d. treatment while an increase to 1.5 mmol/l was observed at 12 weeks during t.i.d treatment. The mean value of S-triglycerides was 1.5 mmol/l at baseline and increased to 1.6 mmol/l at 12 weeks during o.d. treatment while a decrease to 1.4 mmol/l was observed at 12 weeks during t.i.d treatment. The mechanisms behind these small, but still significant changes are unclear and unexpected. As the glucose metabolisms demonstrated an improvement during o.d. treatment the expected changes would have been the reverse for HDL-cholesterol and triglycerides in this treatment group. However, these findings might also be beginning of trend effect due to the comparatively small study population for a lipid effects study. The increase in Triglycerides could however been explained by an increase in insulin sensitivity (e.g. lower HbA1c) due to increased lipolysis and would thus be in line with observed effects on glucose metabolism.

The post-hoc analysis of these lipids in the subgroup of 13 patients with diabetes showed that the difference in change (o.d. vs. t.i.d.) was small (-0.1, i.e. smaller increase or larger decrease in the o.d. group) for all four measured lipid metabolism variables (S-cholesterol, S-LDL, S-HDL and triglycerides). This was not expected to be seen as early as within 3 months and in only 13 subjects and can be considered a major finding in this population of patients. This is also in line with the large beneficial effects on glucose homeostasis seen in these patients. Therefore the diurnal profile produced with the o.d. treatment may be responsible for the significant lipid effects seen in this study.

**Lipids - all subjects - ITT**

| | | **od** | **tid** | **od-tid** | |
|---|---|---|---|---|---|
| | **Baseline** | **12 weeks** | **12 weeks** | **Difference** | **p-value** |
| **S Cholesterol, mmol/L** | 5.3 (1.1) | 5.2 (1.0) | 5.3 (0.9) | 0.0 (0.4) | 0.6729 |
| | 5.1 (3.6; 9.9) | 5.1 (3.5; 9.5) | 5.1 (3.6; 8.4) | 0.0 (-1.0; 1.1) | |
| | n=62 | n=56 | n=56 | n=50 | |
| **S LDL Cholesterol, mmol/L** | 3.1 (1.0) | 3.0 (0.9) | 3.1 (0.9) | 0.0 (0.3) | 0.9716 |
| | 2.9 (1.7; 7.5) | 3.0 (1.6; 7.0) | 3.0 (1.7; 6.2) | 0.0 (-0.8; 0.9) | |
| | n=62 | n=56 | n=56 | n=50 | |
| **S HDL Cholesterol, mmol/L** | 1.4 (0.4) | 1.3 (0.4) | 1.5 (0.4) | -0.1 (0.2) | <.0001 |
| | 1.4 (0.7; 2.4) | 1.3 (0.7; 2.1) | 1.5 (0.8; 2.5) | -0.1 (-0.5; 0.3) | |
| | n=62 | n=56 | n=56 | n=50 | |
| **S Triglycerides, mmol/L** | 1.5 (0.8) | 1.6 (0.9) | 1.4 (0.6) | 0.2 (0.6) | 0.0056 |
| | 1.3 (0.5; 5.2) | 1.4 (0.5; 5.1) | 1.3 (0.5; 3.0) | 0.1 (-1.6; 2.1) | |
| | n=61 | n=56 | n=56 | n=50 | |

### Lipids - diabetics only-ITT

The post-hoc analysis of these lipids in the subgroup of 13 patients with diabetes showed that the difference in change (o.d. vs. t.i.d.) was small. The small reduction in LDL-cholesterol was seen only in the diabetic population and not in the whole study group.

| | | **od** | **tid** | **od-tid** | |
|---|---|---|---|---|---|
| | **Baseline** | **12 weeks** | **12 weeks** | **Difference** | **p-value** |
| **S_Cholesterol, mmol/L** | 4.6 (0.5) | 4.5 (0.5) | 4.8 (0.4) | -0.1 (0.2) | 0.1328 |
| | 4.7 (3.7; 5.3) | 4.4 (3.9; 5.4) | 4.9 (4.0; 5.4) | -0.1 (-0.6; 0.2) | |
| | n=13 | n=11 | n=12 | n=10 | |
| **S_LDL Cholesterol, mmol/L** | 2.5 (0.4) | 2.4 (0.5) | 2.7 (0.5) | -0.1 (0.1) | 0.0117 |
| | 2.6 (1.8; 3.1) | 2.3 (1.6; 3.3) | 2.6 (1.7; 3.5) | -0.1 (-0.3; 0.1) | |
| | n=13 | n=11 | n=12 | n=10 | |
| **S_HDL Cholesterol, mmol/L** | 1.3 (0.3) | 1.3 (0.4) | 1.4 (0.4) | -0.1 (0.1) | 0.0547 |
| | 1.2 (0.7; 1.9) | 1.4 (0.8; 1.8) | 1.4 (0.8; 2.1) | -0.1 (-0.3; 0.1) | |
| | n=13 | n=11 | n=12 | n=10 | |
| **S_Triglycerides, mmol/L** | 1.5 (0.5) | 1.2 (0.6) | 1.5 (0.6) | -0.1 (0.3) | 0.1953 |
| | 1.6 (0.7; 2.2) | 1.2 (0.6; 2.4) | 1.5 (0.7; 2.6) | -0.2 (-0.7; 0.5) | |
| | n=12 | n=11 | n=12 | n=10 | |

### Bone formation and resorption markers

There was an increase in two serum bone formation markers, PINP and osteocalcin indicating a favorable effect on bone metabolism, in particular as patients receiving glucocorticoid replacement therapy have been shown to have reduced bone mass and bone mineral content. One possible mechanism is the decreased plasma cortisol levels or another possibility is the diurnal profile produced with o.d. administration. It is well-known that resorption markers usually precede formation markers at increased bone remodeling.

| | | **od** | **tid** | | **od-tid** |
|---|---|---|---|---|---|
| | **Baseline** | **12 weeks** | **12 weeks** | **Difference** | **p-value** |
| **S_PINP,µg/L** | 57.3 (28.5) | 64.3 (35.0) | 56.5 (29.3) | 6.0 (15.6) | 0.0047 |
| | 49.4 (15.6; 174.0) | 54.0 (23.7; 229.0) | 50.0 (18.6; 167.0) | 6.0 (-25.7; 62.0) | |
| | n=62 | n=57 | n=57 | n=52 | |
| **S**_**Osteocalcin, µg/L** | 11.4(5.6) | 13.5 (6.5) | 12.6 (5.4) | 0.7 (4.5) | 0.2787 |
| | 11.4 (0.1; 25.2) | 13.9 (0.6; 30.6) | 12.6 (1.4; 24.6) | 0.8 (-11.7; 11.7) | |
| | n=62 | n=57 | n=57 | n=52 | |

### Target profile

Fig. 12 shows that the tested treatment fulfills the criteria set forth for the target profile. Figs. 13 shows the mean PK profile for once daily treatment compared with conventional therapy and fig. 14 shows a comparison of the once daily treatment compared with normal cortisol profile.

### References

1. Mason AS, Meade TW, Lee JA, Morris JN 1968 Epidemiological and clinical picture of Addison's disease. Lancet 2:744-747
2. Rosen T, Bengtsson B-Å 1990 Premature mortality due to cardiovascular diseases in hypopituitarism. Lancet 336:285-288
3. Tomlinson JW, Holden N, Hills RK, Wheatley K, Clayton RN, Bates AS, Sheppard MC, Stewart PM 2001 Association between premature mortality and hypopituitarism. West Midlands Prospective Hypopituitary Study Group. Lancet 357:425-431
4. Mills JL, Schonberger LB, Wysowski DK, Brown P, Durako SJ, Cox C, Kong F, Fradkin JE 2004 Long-term mortality in the United States cohort of pituitary-derived growth hormone recipients. J Pediatr 144:430-436
5. Bergthorsdottir R, Leonsson-Zachrisson M, Oden A, Johannsson G 2006 Premature mortality in patients with Addison's disease: a population-based study. J Clin Endocrinol Metab 91:4849-4853
6. Bensing S, Brandt L, Tabaroj F, Sjoberg O, Nilsson B, Ekbom A, Blomqvist P, Kampe O 2008 Increased death risk and altered cancer incidence pattern in patients with isolated or combined autoimmune primary adrenocortical insufficiency. Clin Endocrinol (Oxf) 69:697-704
7. Peacey SR, Guo CY, Robinson AM, Price A, Giles MA, Eastell R, Weetman AP 1997 Glucocorticoid replacement therapy: are patients over treated and does it matter? Clin Endocrinol (Oxf) 1997 Mar;46(3):255-61 46:255-261
8. Dallman MF, Akana SF, Bhatnagar S, Bell ME, Strack AM 2000 Bottomed out: metabolic significance of the circadian trough in glucocorticoid concentrations. Int J Obes Relat Metab Disord 24 Suppl 2:S40-46
9. Dallman MF, la Fleur SE, Pecoraro NC, Gomez F, Houshyar H, Akana SF 2004 Minireview: glucocorticoids--food intake, abdominal obesity, and wealthy nations in 2004. Endocrinology 145:2633-2638
10. Groves RW, Toms GC, Houghton BJ, Monson JP 1988 Corticosteroid replacement therapy: twice or thrice daily? J R Soc Med 81:514-516
11. Lovas K, Husebye ES 2007 Continuous subcutaneous hydrocortisone infusion in Addison's disease. Eur J Endocrinol 157:109-112
12. Suliman AM, Freaney R, Smith TP, McBrinn Y, Murray B, McKenna TJ 2003 The impact of different glucocorticoid replacement schedules on bone turnover and insulin sensitivity in patients with adrenal insufficiency. Clin Endocrinol (Oxf) 59:380-387
13. McConnell EM, Bell PM, Ennis C, Hadden DR, McCance DR, Sheridan B, Atkinson AB 2002 Effects of low-dose oral hydrocortisone replacement versus short-term reproduction of physiological serum cortisol concentrations on insulin action in adult-onset hypopituitarism. Clin Endocrinol (Oxf) 56:195-201
14. Kraan GP, Dullaart RP, Pratt JJ, Wolthers BG, Drayer NM, De Bruin R 1998 The daily cortisol production reinvestigated in healthy men. The serum and urinary cortisol production rates are not significantly different. J Clin Endocrinol Metab 83:1247-1252
15. Esteban NV, Loughlin T, Yergey AL, Zawadzki JK, Booth JD, Winterer JC, Loriaux DL 1991 Daily cortisol production rate in man determined by stable isotope dilution/mass spectrometry. J Clin Endocrinol Metab 72:39-45
16. Filipsson H, Monson JP, Koltowska-Haggstrom M, Mattsson A, Johannsson G 2006 The impact of glucocorticoid replacement regimens on metabolic outcome and comorbidity in hypopituitary patients. J Clin Endocrinol Metab 91:3954-3961
17. Reynolds RM, Stewart PM, Seckl JR, Padfield PL 2006 Assessing the HPA axis in patients with pituitary disease: a UK survey. Clin Endocrinol (Oxf) 64:82-85
18. Peace KA, Orme SM, Sebastian JP, Thompson AR, Barnes S, Ellis A, Belchetz PE 1997 The effect of treatment variables on mood and social adjustment in adult patients with pituitary disease. Clin Endocrinol (Oxf) 46:445-450
19. Dunne FP, Elliot P, Gammage MD, Stallard T, Ryan T, Sheppard MC, Stewart PM 1995 Cardiovascular function and glucocorticoid replacement in patients with hypopituitarism. Clin Endocrinol (Oxf) 43:623-629
20. Danilowicz K, Bruno OD, Manavela M, Gomez RM, Barkan A 2008 Correction of cortisol overreplacement ameliorates morbidities in patients with hypopituitarism: a pilot study. Pituitary 11:279-285

## Claims

1. A dosage form for use in a method of treating a subject suffering from a diminished or disrupted endogenous glucocorticoid secretory pattern, wherein said dosage form is administered once daily, and wherein said subject also suffers from diabetes mellitus, wherein said dosage form is prepared from a composition comprising
(i) 1.5 wt% to 6.5 wt% of hydrocortisone
(ii) 15 wt% to 25 wt% polymer or binder
(iii) 30 wt% to 40 wt% filler
(iv) 0.1 wt% to 0.5 wt% glidant
(v) 0.1 wt% to 0.5 wt% lubricant
(vi) 1 wt% to 5 wt% of film coating system
(vii) 25 wt% to 40 wt% solvent
wherein the percentages are given as % of the total weight of all ingredients used in the manufacture of the composition, wherein the solvent is wholly evaporated during the manufacturing process and wherein the dosage form comprises a first immediate release part in the coating and a second extended release part in the core, and further wherein the first immediate release part contains about 20-30% of the total amount of hydrocortisone and the second extended release part contains about 70-80% of the total amount of hydrocortisone.

2. A dosage form for use according to claim 1, wherein the filler is selected from calcium phosphate, dibasic dihydrate calcium sulphate, cellulose, microcrystalline cellulose, powdered cellulose, silicified microcrystalline ethylcellulose, lactose, sodium chloride, sorbitol, starch or any combinations thereof.

3. A dosage form for use according to any of claims 1-2, wherein the glidant or lubricant is selected from stearic acid, magnesium stearate, calcium stearate or other metallic stearate, talc, waxes and glycerides, light mineral oil, PEG, glyceryl behenate, colloidal silica, hydrogenated vegetable oils, corn starch, sodium stearyl fumarate, polyethylene glycols, alkyl sulfates, sodium benzoate, sodium acetate, talc or any combinations thereof.

4. A dosage form for use according to any of claims 1-3, wherein the film coating system comprises methylcellulose, hydroxypropylmethylcellulose, hydroxypropylcellulose, acrylic polymers, ethylcellulose, cellulose acetate phthalate, polyvinyl acetate phthalate, hydroxypropyl methylcellulose phthalate, polyvinylalcohol, sodium carboxymethylcellulose, cellulose acetate, cellulose acetate phthalate, gelatin, methacrylic acid copolymer, polyethylene glycol, shellac, sucrose, titanium dioxide, carnauba wax, microcrystalline wax, glyceryl monostearate or zein.

5. A dosage form for use according to any of the preceding claims wherein the dosage form is a coated tablet.

6. A dosage form for use as defined in any of claims 1-5, wherein administration of the dosage form for a period of a least 12 weeks results in a reduction of one or more side-effects generally observed with glucocorticoid therapy.

7. A dosage form for use according to claim 6, wherein the one or more side-effects are selected from the group consisting of: gain of weight, increase in cardiovascular risk factors and metabolic risk factors.

8. A dosage form for use according to any of claims 1-5, wherein the dosage form is for oral administration.

9. A dosage form for use according to any of the preceding claims, wherein the dosage form contains a total dose of hydrocortisone from 1 mg to 30 mg.

10. A dosage form for use according to any of the preceding claims, wherein the dosage form is a coated tablet comprising an extended release tablet core and an immediate release coating surrounding said extended release tablet core.

11. A dosage form for use according to any of the preceding claims, wherein the dosage form is administered in the morning.

## Patentansprüche

1. Dosierungsform zur Verwendung in einem Verfahren zur Behandlung eines Subjekts, das an einem verminderten oder unterbrochenen endogenen Glucocorticoid-Sekretionsmuster leidet, wobei die Dosierungsform einmal täglich verabreicht wird, und wobei das Subjekt ebenfalls an Diabetes mellitus leidet, wobei die Dosierungsform aus einer Zusammensetzung hergestellt wird, die umfasst
(i) 1,5 Gew.-% bis 6,5 Gew.-% Hydrocortison
(ii) 15 Gew.-% bis 25 Gew.-% Polymer oder Bindemittel
(iii) 30 Gew.-% bis 40 Gew.-% Füllstoff
(iv) 0,1 Gew.-% bis 0,5 Gew.-% Gleitmittel
(v) 0,1 Gew.-% bis 0,5 Gew.-% Schmiermittel
(vi) 1 Gew.-% bis 5 Gew.-% Filmbeschichtungssystem
(vii) 25 Gew.-% bis 40 Gew.-% Lösungsmittel
wobei die Prozentsätze als % des Gesamtgewichts aller Bestandteile angegeben sind, die bei der Herstellung der Zusammensetzung verwendet werden, wobei das Lösungsmittel während des Herstellungsverfahrens vollständig verdampft wird und wobei die Dosierungsform in der Beschichtung einen ersten Teil mit sofortiger Freisetzung und im Kern einen zweiten Teil mit verlängerter Freisetzung umfasst, und wobei ferner der erste Teil mit sofortiger Freisetzung etwa 20-30% der Gesamtmenge an Hydrocortison enthält und der zweite Teil mit verlängerter Freisetzung etwa 70-80% der Gesamtmenge an Hydrocortison enthält.

2. Dosierungsform zur Verwendung nach Anspruch 1, wobei der Füllstoff ausgewählt ist aus Calciumphosphat, zweibasigem Calciumsulfat-Dihydrat, Cellulose, mikrokristalliner Cellulose, pulverförmiger Cellulose, silizifierter mikrokristalliner Ethylcellulose, Lactose, Natriumchlorid, Sorbitol, Stärke oder beliebigen Kombinationen davon.

3. Dosierungsform zur Verwendung nach beliebigen der Ansprüche 1-2, wobei das Gleitmittel oder Schmiermittel ausgewählt ist aus Stearinsäure, Magnesiumstearat, Calciumstearat oder anderem Metallstearat, Talk, Wachsen und Glyceriden, Leichtmineralöl, PEG, Glycerylbehenat, kolloidalem Siliciumdioxid, hydrierten Pflanzenölen, Maisstärke, Natriumstearylfumarat, Polyethylenglycolen, Alkylsulfaten, Natriumbenzoat, Natriumacetat, Talk oder beliebigen Kombinationen davon.

4. Dosierungsform zur Verwendung nach beliebigen der Ansprüche 1-3, wobei das Filmbeschichtungssystem Methylcellulose, Hydroxypropylmethylcellulose, Hydroxypropylcellulose, Acrylpolymere, Ethylcellulose, Celluloseacetatphthalat, Polyvinylacetatphthalat, Hydroxypropylmethylcellulosephthalat, Polyvinylalkohol, Natriumcarboxymethylcellulose, Celluloseacetat, Celluloseacetatphthalat, Gelatine, Methacrylsäurecopolymer, Polyethylenglycol, Schellack, Saccharose, Titandioxid, Carnaubawachs, mikrokristallines Wachs, Glycerylmonostearat oder Zein umfasst.

5. Dosierungsform zur Verwendung nach beliebigen der vorhergehenden Ansprüche, wobei die Dosierungsform eine beschichtete Tablette ist.

6. Dosierungsform zur Verwendung, wie in beliebigen der Ansprüche 1-5 definiert, wobei die Verabreichung der Dosierungsform für einen Zeitraum von mindestens 12 Wochen zu einer Verringerung von einer oder mehreren Nebenwirkungen führt, die allgemein bei einer Glucocorticoidtherapie beobachtet werden.

7. Dosierungsform zur Verwendung nach Anspruch 6, wobei die eine oder mehreren Nebenwirkungen ausgewählt sind aus der Gruppe bestehend aus: Gewichtszunahme, Zunahme von kardiovaskulären Risikofaktoren und metabolischen Risikofaktoren.

8. Dosierungsform zur Verwendung nach beliebigen der Ansprüche 1-5, wobei die Dosierungsform für die orale Verabreichung bestimmt ist.

9. Dosierungsform zur Verwendung nach beliebigen der vorhergehenden Ansprüche, wobei die Dosierungsform eine Gesamtdosis an Hydrocortison von 1 mg bis 30 mg enthält.

10. Dosierungsform zur Verwendung nach beliebigen der vorhergehenden Ansprüche, wobei die Dosierungsform eine beschichtete Tablette ist, umfassend einen Tablettenkern mit verlängerter Freisetzung und eine Beschichtung mit sofortiger Freisetzung, die den Tablettenkern mit verlängerter Freisetzung umgibt.

11. Dosierungsform zur Verwendung nach beliebigen der vorhergehenden Ansprüche, wobei die Dosierungsform morgens verabreicht wird.

## Revendications

1. Forme pharmaceutique pour utilisation dans un procédé de traitement d'un sujet souffrant d'un profil sécrétoire de glucocorticoïde endogène réduit ou perturbé, ladite forme pharmaceutique étant administrée une fois par jour, et ledit sujet souffrant en outre de diabète sucré, ladite forme pharmaceutique étant préparée à partir d'une composition comprenant
(i) 1,5 % en poids à 6,5 % en poids d'hydrocortisone
(ii) 15 % en poids à 25 % en poids de polymère ou de liant
(iii) 30 % en poids à 40 % en poids de charge
(iv) 0,1 % en poids à 0,5 % en poids d'agent glissant
(v) 0,1 % en poids à 0,5 % en poids de lubrifiant
(vi) 1 % en poids à 5 % en poids de système de pelliculage
(vii) 25 % en poids à 40 % en poids de solvant
dans laquelle les pourcentages sont exprimés en % du poids total de la totalité des composants utilisés dans la fabrication de la composition, le solvant étant totalement évaporé pendant le processus de fabrication et la forme pharmaceutique comprenant une première partie à libération immédiate d'un enrobage et une deuxième partie à libération prolongée dans le noyau, et la première partie à libération immédiate contenant en outre environ 20 à 30 % de la quantité totale d'hydrocortisone et la deuxième partie à libération prolongée contenant environ 70 à 80 % de la quantité totale d'hydrocortisone.

2. Forme pharmaceutique pour utilisation selon la revendication 1, dans laquelle la charge est choisie parmi le phosphate de calcium, le sulfate de calcium dihydraté dibasique, la cellulose, la cellulose microcristalline, la cellulose pulvérulente, l'éthylcellulose microcristalline silicifiée, le lactose, le chlorure de sodium, le sorbitol, l'amidon ou des combinaisons quelconques de ceux-ci.

3. Forme pharmaceutique pour utilisation selon l'une quelconque des revendications 1 à 2, dans laquelle l'agent glissant ou le lubrifiant est choisi parmi l'acide stéarique, le stéarate de magnésium, le stéarate de calcium ou un autre stéarate métallique, le talc, des cires et des glycérides, l'huile minérale légère, PEG, le béhénate de glycéryle, la silice colloïdale, des huiles végétales hydrogénées, l'amidon de maïs, le fumarate de stéaryle sodique, des polyéthylène glycols, des sulfates d'alkyle, le benzoate de sodium, l'acétate de sodium, le talc ou des combinaisons quelconques de ceux-ci.

4. Forme pharmaceutique pour utilisation selon l'une quelconque des revendications 1 à 3, dans laquelle le système de pelliculage comprend la méthylcellulose, l'hydroxypropylméthylcellulose, l'hydroxypropylcellulose, des polymères acryliques, l'éthylcellulose, l'acétate-phtalate de cellulose, le poly(acétate-phtalate de vinyle), le phtalate d'hydroxypropylméthylcellulose, l'alcool polyvinylique, le carboxyméthylcellulose sodique, l'acétate de cellulose, l'acétate-phtalate de cellulose, la gélatine, un copolymère d'acide méthacrylique, le polyéthylène glycol, la gomme laque, le saccharose, le dioxyde de titane, la cire de carnauba, la cire microcristalline, le monostéarate de glycéryle ou la zéine.

5. Forme pharmaceutique pour utilisation selon l'une quelconque des revendications précédentes, la forme pharmaceutique étant un comprimé enrobé.

6. Forme pharmaceutique pour utilisation telle que définie dans l'une quelconque des revendications 1 à 5, dans laquelle l'administration de la forme pharmaceutique pendant une durée d'au moins 12 semaines conduit à une réduction d'un ou plusieurs effets secondaires généralement observés avec une thérapie par des glucocorticoïdes.

7. Forme pharmaceutique pour utilisation selon la revendication 6, les un ou plusieurs effets secondaires étant choisis dans le groupe constitué de : un gain de poids, une augmentation de facteurs de risque cardiovasculaires et de facteurs de risque métaboliques.

8. Forme pharmaceutique pour utilisation selon l'une quelconque des revendications 1 à 5, la forme pharmaceutique étant pour administration orale.

9. Forme pharmaceutique pour utilisation selon l'une quelconque des revendications précédentes, la forme pharmaceutique contenant une dose totale d'hydrocortisone de 1 mg à 30 mg.

10. Forme pharmaceutique pour utilisation selon l'une quelconque des revendications précédentes, la forme pharmaceutique étant un comprimé enrobé comprenant un noyau de comprimé à libération prolongée et un enrobage à libération immédiate entourant ledit noyau de comprimé à libération prolongée.

11. Forme pharmaceutique pour utilisation selon l'une quelconque des revendications précédentes, la forme pharmaceutique étant administrée le matin.
